# EUROPÄISCHE PATENTSCHRIFT

(11) **EP 1 888 668 B1**
(45) Veröffentlichungstag und Bekanntmachung des Hinweises auf die Patenterteilung: **10.09.2008**
(21) Anmeldenummer: 06755042.6
(22) Anmeldetag: 05.05.2006
(51) Int. Cl.: C08G 77/38, C08G 77/14, C08G 77/26, A61K 8/58, D06M 15/643, C11B 9/00, C11D 3/50

(54) **RIECHSTOFFALKOHOL FREISETZENDES POLYSILOXAN**
ALCOHOL ODOROUS SUBSTANCE-RELEASING POLYSILOXANE
POLYSILOXANE LIBERANT UN ALCOOL DE PARFUM

(30) Priorität: 09.06.2005 US 688695 P; 10.06.2005 DE 102005026796
(43) Veröffentlichungstag der Anmeldung: 20.02.2008
(73) Patentinhaber: Evonik Degussa GmbH, 45128 Essen (DE)
(72) Erfinder: KÖHLE, Hans-Jürgen, 63533 Mainhausen (DE); SALOMON, Thomas, 63628 Bad Soden-Salmünster (DE); SMITH, Ronald Coleman Jr., Chesterfield, Virginia 23832 (US)
(86) Internationale Anmeldenummer: PCT/EP2006/062089
(87) Internationale Veröffentlichungsnummer: WO 2006/131430

(56) Entgegenhaltungen:
- EP-A- 0 878 497
- EP-A- 0 982 023
- EP-A- 1 099 689
- DE-A1- 19 750 706

## Beschreibung

Gegenstand der Erfindung sind Organopolysiloxane, aus denen unter sauren Bedingungen ein Riechstoffalkohol freigesetzt werden kann.

Für Textilbehandlungsmittel und Körperpflegemittel werden Parfümkomponenten benötigt, die auf Textilfasern, Haaren oder Haut über einen längeren Zeitraum haften und dort die Riechstoffe des Parfüms über einen längeren Zeitraum und unter kontrollierten Bedingungen freisetzen. Besonders wünschenswert sind dabei Parfümkomponenten, die ihre Riechstoffe erst dann freisetzen, wenn der Benutzer des Körperpflegemittels oder des mit dem Textilbehandlungsmittels behandelten Kleidungsstück schwitzt und sich durch das Schwitzen unangenehme Körpergerüche bilden. Unangenehme Körpergerüche entstehen dabei in der Regel durch Bakterien, die Bestandteile des Schweißes abbauen. Dieser bakterielle Abbau führt neben der Bildung unangenehm riechender Verbindungen auch zu sauren Abbauprodukten. Deshalb besteht ein Bedarf nach Parfümkomponenten für Textilbehandlungsmittel und Körperpflegemittel, die Riechstoffe in gebundener Form enthalten und diese erst unter sauren Bedingungen freisetzen, so dass eine verstärkte Parfümwirkung unter diesen Bedingungen erzielt werden kann, um entstehende Körpergerüche zu überdecken.

Aus WO 96/38528 sind Betainester von Riechstoffalkoholen bekannt, die in Waschmitteln, Weichspülern oder Reinigungsmitteln eingesetzt werden können und auf Oberflächen, wie Textilfasern haften. Aus diesen Betainestern wird der Riechstoffalkohol durch Hydrolyse freigesetzt, wobei die Freisetzung des Riechstoffalkohols unter sauren Bedingungen nur sehr langsam erfolgt. Erst bei pH-Werten von 7 oder höher wird der Riechstoffalkohol aus den Betainestern freigesetzt.

Auch aus WO 97/36978 ist bekannt, dass Betainester von Riechstoffalkoholen im Sauren hydrolysestabil sind und dass erst bei pH-Werten von mehr als 7 der Riechstoffalkohol durch Hydrolyse freigesetzt wird.

Ebenso im Sauren stabil sind die in EP-A 1 099 689 offenbarten Betainester von Riechstoffalkoholen.

Die gleiche pH-Abhängigkeit der Stabilität von Betainestern ist auch aus DE 35 27 974 für Betainester bekannt, die als Bestandteile von Haarpflegemitteln eingesetzt werden.

Aus US 4,524,018 sind Alkoxysilane bekannt, die einen Riechstoffalkohol ROH in Form einer Si-O-R-Gruppe gebunden enthalten. Aus diesen Verbindungen wird der Riechstoffalkohol langsam freigesetzt. Das Dokument enthält keine Offenbarung zur pH-Abhängigkeit der Freisetzung der Riechstoffalkohole aus den Alkoxysilanen und die offenbarten Alkoxysilane bleiben nicht an Textilfasern, Haut oder Haaren haften, da sie keine positive Ladung aufweisen oder ausbilden können.

EP-A 0 982 023 beschreibt Polysiloxane, die einen Riechstoffalkohol über eine Si-O-R-Gruppe gebunden enthalten, sowie deren Verwendung als Bestandteil von kosmetischen Zubereitungen. In Gegenwart von Essigsäure wird aus den Polysiloxanen der Riechstoffalkohol langsam freigesetzt. Die offenbarten Polysiloxane zeigen aber keine ausreichende Haftung an Oberflächen mit negativer Oberflächenladung, wie Textilfasern, Haut oder Haaren, da sie keine positive Ladung aufweisen oder ausbilden können.

Es wurde nun überraschend gefunden, dass Organopolysiloxane, die einen Riechstoffalkohol Y-OH über eine Betainester-Gruppe der Struktur -N⁺R¹R²-CH₂-C(O)OY gebunden enthalten, den Riechstoffalkohol unter sauren Bedingungen abspalten und im neutralen und schwach basischen Bereich unerwartet hydrolysestabil sind. Diese Organopolysiloxane zeigen außerdem eine gute Haftung auf Textilfasern, Haut oder Haaren, die sie als Parfümkomponenten für Textilbehandlungsmittel und Körperpflegemittel geeignet macht.

Gegenstand der Erfindung sind demnach Organopolysiloxane, aus denen ein Riechstoffalkohol freigesetzt werden kann, dadurch gekennzeichnet, dass das Organopolysiloxan mindestens eine funktionelle Gruppe Z der Struktur

-N⁺R¹R²-CH₂-C (O) OY A⁻

aufweist, worin
R¹, R² unabhängig voneinander ausgewählt sind aus C₁₋₃₀ Alkyl und Hydroxyethyl,
Y der Rest eines Riechstoffalkohols Y-OH ist und
A⁻ das Anion einer physiologisch verträglichen Säure HA ist.

Gegenstand der Erfindung sind außerdem Textilbehandlungsmittel und Körperpflegemittel, die mindestens ein Organopolysiloxan der oben genannten Struktur enthalten, sowie Waschmittel, die mindestens ein Tensid und mindestens ein Organopolysiloxan der oben genannten Struktur enthalten und Duftspüler, die mindestens ein Organopolysiloxan der oben genannten Struktur in Form einer wässrigen Dispersion enthalten.

Organopolysiloxane im Sinne der Erfindung sind Verbindungen, die mindestens drei Siliziumatome enthalten, die über Si-O-Si-Einheiten miteinander verbunden sind und in denen mehr als die Hälfte der Siliziumatome mindestens einen über ein Kohlenstoffatom an das Siliziumatom gebundenen Rest aufweist. Die erfindungsgemäßen Organopolysiloxane enthalten vorzugsweise von 3 bis 200 und besonders bevorzugt von 5 bis 100 Siliziumatome. Vorzugsweise weist in den erfindungsgemäßen Organopolysiloxanen mehr als 50 % und besonders bevorzugt mehr als 80 % der Siliziumatome zwei über Kohlenstoffatome an das Siliziumatom gebundene Reste auf. Die über Kohlenstoffatome an Siliziumatome gebundenen Reste können C₁₋₃₀ Alkylreste, C₂₋₃₀ Alkenylreste oder C₆₋₃₀ Arylreste sein, die weitere Substituenten aufweisen können. Die über Kohlenstoffatome an Siliziumatome gebundenen Reste sind vorzugsweise Methylgruppen oder Phenylgruppen und besonders bevorzugt Methylgruppen.

Die erfindungsgemäßen Organopolysiloxane weisen mindestens eine Betainestergruppe der Struktur

-N⁺R¹R²-CH₂-C(O)OY A⁻

auf, die kovalent an das Polysiloxan gebunden ist und für die
R¹, R² unabhängig voneinander ausgewählt sind aus C₁₋₃₀ Alkyl und Hydroxyethyl,
Y der Rest eines Riechstoffalkohols Y-OH ist und
A⁻ das Anion einer physiologisch verträglichen Säure HA ist.

In einer bevorzugten Ausführungsform der Erfindung sind die Betainestergruppen über Hydroxyethylen-Brücken mit Siliziumatomen des Organopolysiloxans verknüpft und das Organopolysiloxan weist die Struktur

R³R⁴R⁵Si-O-CH₂-CH₂-N⁺R¹R²-CH₂-C(O)OY A⁻

auf, wobei
R³ ein über ein Sauerstoffatom gebundener Polysiloxanrest ist,
R⁴ eine C₁₋₃₀ Alkylgruppe oder Phenyl ist,
R⁵ = R³ oder R⁴ ist und
R¹, R², Y und A⁻ die oben genannte Bedeutung haben.

In einer besonders bevorzugten Ausführungsform der Erfindung sind an die terminalen Siliziumatome eines Polydimethylsiloxans mit 3 bis 200 Siliziumatomen Betainestergruppen über Hydroxyethylen-Brücken gebunden. In dieser besonders bevorzugten Ausführungsform weist das Organopolysiloxane die Struktur

R⁶-O-[Si(CH₃)₂-O]ₙ-R⁶

auf, wobei
n = 3-200 ist,
R⁶ = -CH₂-CH₂-N⁺(CH₃)₂-CH₂-C(O)OY A⁻ ist und
Y und A⁻ die oben genannte Bedeutung haben.

Riechstoffalkohole Y-OH im Sinne der Erfindung sind alle Verbindungen, die einen Geruch aufweisen und die eine an ein Kohlenstoffatom gebundene Hydroxylgruppe aufweisen, wobei an dieses Kohlenstoffatom keine weiteren Atome außer Wasserstoff und Kohlenstoff gebunden sind. Vorzugsweise enthalten die erfindungsgemäßen Organopolysiloxane Riechstoffalkohole Y-OH gebunden, die als Bestandteile von Parfümzubereitungen verwendet werden.

Besonders bevorzugt enthalten die erfindungsgemäßen Organopolysiloxane Riechstoffalkohole Y-OH gebunden, die ausgewählt sind aus der Reihe 4-Allyl-2-methoxyphenol (Eugenol), 3-(2-Bornyloxy)-2-methyl-1-propanol, 2-tert-Butylcyclohexanol, 4-tert-Butylcyclohexanol, Benzylalkohol, 1-Decanol, 9-Decen-1-ol, Dihydroterpineol, 2,4-Dimethyl-4-cyclohexen-1-ylmethanol, 2,4-Dimethylcyclohexylmethanol, 2,6-Dimethyl-2-heptanol, 2,6-Dimethyl-4-heptanol, 3a,4,5,6,7,7a-Hexahydro-2,4-dimethyl-4,7-methano[1H]inden-5-ol, 3,7-Dimethyl-1,6-nonadien-3-ol, 2,6-Dimethyl-2,7-octadien-6-ol (Linalool), cis-3,7-Dimethyl-2,6-octadien-1-ol (Nerol), trans-3,7-Dimethyl-2,6-octadien-1-ol (Geraniol), 3,7-Dimethyl-1,7-octandiol, 3,7-Dimethyl-1-octanol (Tetrahydrogeraniol), 2,6-Dimethyl-2-octanol (Tetrahydromyrcenol), 3,7-Dimethyl-3-octanol (Tetrahydrolinalool), 2,6-Dimethyl-7-octen-2-ol (Dihydromyrcenol), 3,7-Dimethyl-6-octen-1-ol (Citronellol), 2,2-Dimethyl-3-(3-methylphenyl)-1-propanol, 2,2-Dimethyl-3-phenyl-1-propanol, 2-Ethoxy-4-methoxymethylphenol, 2-Ethyl-4-(2,2,3-trimethyl-3-cyclopenten-1-yl)-2-buten-1-ol, cis-3-Hexen-1-ol, 4-(4-Hydroxy-3-methoxyphenyl)-2-butanon, 1-Hydroxy-2-(1-methyl-1-hydroxyethyl)-5-methylcyclohexan, 3-(Hydroxymethyl)-2-nonanon, 4-(4-Hydroxy-4-methylpentyl)-3-cyclohexen-1-carboxaldehyde, Isoborneol, 3-Isocamphylcyclohexanol, 2-Isopropenyl-5-methylcyclohexanol (Isopulegol), 1-Isopropyl-4-methylcyclohex-3-enol (Terpinenol), 4-Isopropylcyclohexanol, 1-(4-Isopropylcyclohexyl)-ethanol, 4-Isopropylcyclohexylmethanol, 2-Isopropyl-5-methylcyclohexanol (Menthol), 2-Isopropyl-5-methylphenol (Thymol), 5-Isopropyl-2-methylphenol (Carvacrol), 2-(4-Methyl-3-cyclohexenyl)-2-propanol (Terpineol), 2-(4-Methylcyclohexyl)-2-propanol (Dihydroterpineol), 4-Methoxybenzylalcohol, 2-Methoxy-4-methylphenol, 3-Methoxy-5-methylphenol, 1-Methoxy-4-propenylbenzol (Anethol), 2-Methoxy-4-propenylphenol (Isoeugenol), 4-Methyl-3-decen-5-ol, 2-Methyl-6-methylene-7-octen-2-ol (Myrcenol), 3-Methyl-4-phenyl-2-butanol, 2-(2-Methylphenyl)-ethanol, 2-Methyl-4-phenyl-1-pentanol, 3-Methyl-5-phenyl-1-pentanol, 2-Methyl-1-phenyl-2-propanol, (1-Methyl-2-(1,2,2-trimethylbicyclo[3.1.0]hex-3-ylmethyl)-cyclopropyl)-methanol, 3-Methyl-4-(2,2,6-trimethylcyclohexen-1-yl)-2-butanol, 2-Methyl-4-(2,2,3-trimethyl-3-cyclopenten-1-yl)-2-buten-1-ol, (3-methyl-1-(2,2,3-trimethyl-3-cyclopentenyl)-3-cyclohexen-1-yl)-methanol, 3-Methyl-5-(2,2,3-trimethyl-3-cyclopenten-1-yl)-4-penten-2-ol, 2-Methyl-2-vinyl-5-(1-hydroxy-1-methylethyl)-tetrahydrofuran, trans,cis-2,6-Nonadienol, 1-Nonanol, Nopol, 1,2,3,4,4a,5,6,7-Octahydro-2,5,5-trimethyl-2-naphthol, 1-Octanol, 3,4,5,6,6-Pentamethyl-2-heptanol, 2-Phenylethanol, 2-Phenylpropanol, 3-Phenylpropanol (Hydrozimtalkohol), 3-Phenyl-2-propen-1-ol (Zimtalkohol), 4-(5,5,6-Trimethylbicyclo[2.2.1]hept-2-yl)-cyclohexan-1-ol, 3,5,5-Trimethylcyclohexanol, 2,4,6-Trimethyl-4-cyclohexen-1-ylmethanol, 5-(2,2,3-Trimethyl-3-cyclopentenyl)-3-methylpentan-2-ol, 3,7,11-Trimethyl-2,6,10-dodecatrien-1-ol (Farnesol), 3,7,11-Trimethyl-1,6,10-dodecatrien-3-ol (Nerolidol), 3,5,5-Trimethyl-1-hexanol (Isononanol), 1-Undecanol, 10-Undecen-1-ol und Vetiverol.

Anionen A⁻ von physiologisch verträglichen Säuren HA im Sinne der Erfindung sind alle Anionen, die keine ätzende oder ausgeprägt reizende Wirkung auf menschliche Haut haben. Vorzugsweise werden die Anionen A⁻ aus der Reihe Chlorid, Bromid, Methylsulfat, Ethylsulfat, Sulfat, Nitrat, Phosphat oder Hydrogenphosphat gewählt.

Die erfindungsgemäßen Organopolysiloxane lassen sich durch Umsetzung von Organopolysiloxanen, die eine tertiäre Aminogruppe enthalten, mit einem Chloressigsäureester eines Riechstoffalkohols erhalten. Chloressigsäureester von Riechstoffalkoholen sind in bekannter Weise durch die Umsetzung von Chloressigsäurechlorid mit dem Riechstoffalkohol zugänglich, wobei die Umsetzung vorzugsweise in Gegenwart einer Base durchgeführt wird, die den bei der Reaktion frei gesetzten Chlorwasserstoff bindet.

Organopolysiloxane, die eine tertiäre Aminogruppe enthalten, sind durch eine Reihe von bekannten Verfahren zugänglich. Ein allgemeiner Weg zur Herstellung dieser Verbindungen ist die Umsetzung eines sekundären Amins mit einem Organopolysiloxan, das eine geeignete Austrittsgruppe enthält, in einer nucleophilen Substitutionsreaktion. Eine spezielle Ausführungsform dieser Reaktion ist die Umsetzung eines Organopolysiloxans, das einen Rest mit einer Epoxidgruppierung enthält, mit einem sekundären Amin unter Ringöffnung des Epoxids und Bildung eines beta-Aminoalkohols.

Die bevorzugten Organopolysiloxane, in denen die Betainestergruppen über Hydroxyethylen-Brücken mit Siliziumatomen des Organopolysiloxans verknüpft sind, werden vorzugsweise durch Umsetzung eines Organopolysiloxans, das mindestens eine Si-H-Gruppe aufweist, mit einem Ethanolamin der Struktur HO-CH₂-CH₂-NR¹R² und nachfolgende Umsetzung mit einem Chloressigsäureester eines Riechstoffalkohols erhalten, wobei R¹ und R² die zuvor genannte Bedeutung haben.
R³R⁹R⁵Si-H + HO-CH₂-CH₂-NR¹R² → R³R⁴R⁵Si-O-CH₂-CH₂-NR¹R²
R³R⁴R⁵Si-O-CH₂-CH₂-NR¹R² + ClCH₂-C(O)OY →
R³R⁴R⁵Si-O-CH₂-CH₂-N⁺R¹R²-CH₂-C(O)OY Cl⁻

Die erfindungsgemäßen Organopolysiloxane zeigen in wässriger Dispersion eine hohe Stabilität gegen Hydrolyse und sind in der Lage, aus wässriger Dispersion auf Textilfasern, Haut oder Haare aufzuziehen. Der Begriff wässrige Dispersion im Sinn der Erfindung beschränkt sich dabei nicht auf Dispersionen fester Organopolysiloxane, sondern umfasst auch Emulsionen flüssiger Organopolysiloxane, sowie Mikroemulsionen und micellare Lösungen. Die erfindungsgemäßen Organopolysiloxane eignen sich damit als Bestandteile von Textilbehandlungsmitteln oder Körperpflegemitteln und können solchen Mitteln die Eigenschaft vermitteln, dass mit dem Textilbehandlungsmittel behandelte Textilien oder mit dem Körperpflegemittel behandelte Haare oder Hautpartien einen lange anhaltenden Duft aufweisen, der von dem aus dem Organopolysiloxan verzögert freigesetzten Riechstoffalkohol stammt.

Bevorzugt sind dabei erfindungsgemäße Organopolysiloxane, die in wässriger Dispersion den Riechstoffalkohol Y-OH bei einem pH-Wert von 3 schneller freisetzen als bei einem pH-Wert von 9. Die raschere Freisetzung des Riechstoffalkohols im Sauren ist dabei unerwartet und war nicht vorhersehbar, da die aus dem Stand der Technik bekannten Betainester von Riechstoffalkoholen alle bei einem pH-Wert von 3 den Riechstoffalkohollangsamer freisetzen als bei einem pH-Wert von 9.

Mit diesen bevorzugten Organopolysiloxanen lassen sich Textilbehandlungsmittel oder Körperpflegemittel bereit stellen, bei denen mit dem Textilbehandlungsmittel behandelte Textilien oder mit dem Körperpflegemittel behandelte Haare oder Hautpartien einen stärkeren Duft durch Freisetzung des Riechstoffalkohols entwickeln, wenn sie durch Schweiß benetzt werden und es durch bakteriellen Abbau von Inhaltsstoffen des Schweißes zu einer Absenkung des pH-Werts kommt. Textilbehandlungsmittel oder Körperpflegemittel, die solche Organopolysiloxane enthalten, können damit wirkungsvoll die Bildung von störenden Gerüchen überdecken, die durch einen bakteriellen Abbau von Inhaltsstoffen des Schweißes entstehen.

Die erfindungsgemäßen Textilbehandlungsmittel enthalten mindestens ein erfindungsgemäßes Organopolysiloxan und sind vorzugsweise für eine Behandlung von Textilien mit einer wässrigen Lösung oder Dispersion des Textilbehandlungsmittels vorgesehen, aus der das bzw. die erfindungsgemäßen Organopolysiloxane auf die Textilfasern aufziehen. Die erfindungsgemäßen Organopolysiloxane bewirken neben der gezielten Freisetzung des Riechstoffalkohols auch einen weicheren Griff des Gewebes, auf dessen Fasern das Organopolysiloxan aufgezogen ist. Die erfindungsgemäßen Textilbehandlungsmittel weisen deshalb auch textilweichmachende Eigenschaften auf.

Die erfindungsgemäßen Textilbehandlungsmittel können eine feste Form haben, beispielsweise die Form von Pulvern, Granulaten oder Formkörpern, wie Extrudaten, Pellets, Briketts oder Tabletten. Textilbehandlungsmittel in fester Form können auch flüssige erfindungsgemäße Organopolysiloxane enthalten, die auf geeignete Trägermaterialien aufgebracht sind. Als Trägermaterialien für flüssige erfindungsgemäße Organopolysiloxane werden dabei vorzugsweise poröse feste Bestandteile des Textilbehandlungsmittels verwendet, die im Textilbehandlungsmittel eine weitere Funktion erfüllen. Textilbehandlungsmittel in Form von Formkörpern, die durch Verfahren der Pressagglomeration, wie Extrusion, Brikettieren oder Tablettieren hergestellt werden, können zusätzlich Binder enthalten um die Festigkeit der Formkörper zu verbessern. Vorzugsweise enthalten die Textilbehandlungsmittel aber keinen zusätzlichen Binder und eine funktionelle Komponente des Textilbehandlungsmittels, vorzugsweise ein nichtionisches Tensid, wirkt als Bindemittel.

In alternativen Ausführungsformen können die erfindungsgemäßen Textilbehandlungsmittel auch flüssig sein oder in Form eines Gels vorliegen. In diesen Ausführungsformen enthält das Textilbehandlungsmittel das erfindungsgemäße Organopolysiloxan vorzugsweise in gelöster oder dispergierter Form, wobei der Begriff dispergierte Form sowohl Dispersionen fester Organopolysiloxane, als auch Emulsionen flüssiger Organopolysiloxane, sowie Mikroemulsionen und micellare Lösungen umfasst.

Textilbehandlungsmittel in flüssiger Form können isotrope, thixotrope, pseudoplastische oder strukturviskose rheologische Eigenschaften aufweisen. Zur Einstellung der rheologischen Eigenschaften können flüssige oder gelförmige Textilbehandlungsmittel als Additive quellende Tone, insbesondere Montmorillonite, gefällte oder pyrogene Kieselsäuren, pflanzliche Gummi, insbesondere Xanthane oder polymere Gelierungsmittel, wie Carboxylgruppen enthaltende Vinylpolymere enthalten. Diese Additive verleihen einerseits dem Textilbehandlungsmittel die gewünschte Viskosität und halten andererseits in dem Textilbehandlungsmittel dispergierte unlösliche Bestandteile in dispergierter Form.

Eine bevorzugte Ausführungsform der erfindungsgemäßen Textilbehandlungsmittel sind Waschmittel, die mindestens ein Tensid und mindestens ein erfindungsgemäßes Organopolysiloxan enthalten. Waschmittel im Sinne der Erfindung sind dabei alle Zubereitungen, die zum Reinigen von Textilien in einer wässrigen Waschflotte geeignet sind.

Als Tenside eignen sich für die erfindungsgemäßen Waschmittel vor allem anionische, nichtionische und kationische Tenside.

Geeignete anionische Tenside sind zum Beispiel Tenside mit Sulfonatgruppen, vorzugsweise Alkylbenzolsulfonate, Alkansulfonate, alpha-Olefinsulfonate, alpha-Sulfofettsäureester oder Sulfosuccinate. Bei Alkylbenzolsulfonaten werden solche mit einer geradkettigen oder verzweigten Alkylgruppe mit 8 bis 20 Kohlenstoffatomen, insbesondere mit 10 bis 16 Kohlenstoffatomen bevorzugt. Bevorzugte Alkansulfonate sind solche mit gradkettigen Alkylketten mit 12 bis 18 Kohlenstoffatomen. Bei alpha-Olefinsulfonaten werden vorzugsweise die Reaktionsprodukte der Sulfonierung von alpha-Olefinen mit 12 bis 18 Kohlenstoffatomen eingesetzt. Bei den alpha-Sulfofettsäureestern werden Sulfonierungsprodukte von Fettsäureestern aus Fettsäuren mit 12 bis 18 Kohlenstoffatomen und kurzkettigen Alkoholen mit 1 bis 3 Kohlenstoffatomen bevorzugt. Als anionische Tenside eignen sich auch Tenside mit einer Sulfatgruppe im Molekül, vorzugsweise Alkylsulfate und Ethersulfate. Bevorzugte Alkylsulfate sind solche mit geradkettigen Alkylresten mit 12 bis 18 Kohlenstoffatomen. Geeignet sind außerdem beta-verzweigte Alkylsulfate und in der Mitte der längsten Alkylkette ein- oder mehrfach alkylsubstituierte Alkylsulfate. Bevorzugte Ethersulfate sind die Alkylethersulfate, die durch Ethoxylierung von linearen Alkoholen mit 12 bis 18 Kohlenstoffatomen mit 2 bis 6 Ethylenoxideinheiten und anschließende Sulfatierung erhalten werden. Als anionische Tenside können schließlich auch Seifen verwendet werden, wie zum Beispiel Alkalimetallsalze von Laurinsäure, Myristinsäure, Palmitinsäure, Stearinsäure und/oder natürlichen Fettsäuregemischen, wie zum Beispiel Kokos-, Palmkern- oder Talgfettsäuren.

Als nicht-ionische Tenside eignen sich zum Beispiel alkoxylierte Verbindungen, insbesondere ethoxylierte und propoxylierte Verbindungen. Besonders geeignet sind Kondensationsprodukte von Alkylphenolen oder Fettalkoholen mit 1 bis 50 mol, vorzugsweise 1 bis 10 mol Ethylenoxid und/oder Propylenoxid. Ebenfalls geeignet sind Polyhydroxyfettsäureamide, in denen am Amidstickstoff ein organischer Rest mit einer oder mehreren Hydroxylgruppen, welche auch alkoxyliert sein können, gebunden ist. Ebenfalls als nicht-ionische Tenside geeignet sind Alkylglycoside mit einer geradkettigen oder verzweigten Alkylgruppe mit 8 bis 22 Kohlenstoffatomen, insbesondere mit 12 bis 18 Kohlenstoffatomen und einem Mono- oder Diglycosidrest, der vorzugsweise von Glucose abgeleitet ist.

Geeignete kationische Tenside sind beispielsweise mono- und dialkoxylierte quaternäre Amine mit einem am Stickstoff gebundenen C₆- bis C₁₈-Alkylrest und ein oder zwei Hydroxyalkylgruppen.

Die erfindungsgemäßen Waschmittel können neben mindestens einem erfindungsgemäßen Organopolysiloxan und mindestens einem Tensid als weitere Komponenten beispielsweise noch Builder, alkalische Komponenten, Bleichmittel, Bleichaktivatoren, Enzyme, chelatisierende Komplexbildner, Vergrauungsinhibitoren, Schauminhibitoren, optische Aufheller, Duftstoffe und Farbstoffe enthalten.

Die erfindungsgemäßen Waschmittel können Builder enthalten, die in der Lage sind, bei der Verwendung im Wasser gelöste Calcium- und Magnesiumionen zu binden. Geeignete Builder sind Alkalimetallphosphate und Alkalimetallpolyphosphate, insbesondere Pentanatriumtriphosphat; wasserlösliche und wasserunlösliche Natriumsilikate, insbesondere Schichtsilikate der Formel Na₅Si₂O₅; Zeolithe der Strukturen A, X und/oder P; sowie Trinatriumcitrat. Zusätzlich zu den Buildern können außerdem organische Cobuilder, wie zum Beispiel Polyacrylsäure, Polyasparaginsäure und/oder Acrylsäure-Copolymere mit Methacrylsäure, Acrolein oder sulfonsäurehaltigen Vinylmonomeren, sowie deren Alkalimetallsalze verwendet werden.

Die erfindungsgemäßen Waschmittel enthalten außerdem in der Regel alkalische Komponenten, die bei bestimmungsgemäßer Anwendung in der Waschflotte, beziehungsweise der wässrigen Reinigungsmittellösung einen pH-Wert im Bereich von 8 bis 12 bewirken. Als alkalische Komponenten sind vor allem Natriumcarbonat, Natriumsesquicarbonat, Natriummetasilikat und andere lösliche Alkalimetallsilikate geeignet.

Die erfindungsgemäßen Waschmittel können weiterhin Bleichmittel enthalten, wie zum Beispiel Alkalimetallperborate, Alkalimetallcarbonatperhydrate, Alkalimetallpersilicate, Alkalimetallpersulfate, Alkalimetallperoxophosphate, Alkalimetallperoxopyrophosphate, Diacylperoxide, aromatische Peroxysäuren und aliphatische Peroxysäuren. Bevorzugte Bleichmittel sind Natriumperborat-Tetrahydrat, Natriumperborat-Monohydrat, Natriumcarbonat-Perhydrat, Peroxylaurinsäure, Peroxystearinsäure, epsilon-Phthalimidoperoxycarbonsäure, 1,12-Diperoxydodecanedisäure, 1,9-Diperoxyazelainsäure und 2-Decyldiperoxybutane-1,4-disäure. Besonders bevorzugt sind Natriumperborat-Tetrahydrat, Natriumperborat-Monohydrat und Natriumcarbonat-Perhydrat, insbesondere Natriumcarbonat-Perhydrat. Geeignetes Natriumcarbonat-Perhydrat zur Verwendung in flüssigen Waschmitteln ist aus WO 2004/056955 bekannt.

Die erfindungsgemäßen Waschmittel können zusätzlich zu Bleichmitteln auch noch Bleichaktivatoren enthalten. Als Bleichaktivatoren eignen sich für die erfindungsgemäßen Waschmittel vor allem Verbindungen mit einer oder mehreren perhydrolysefähigen an Stickstoff oder an Sauerstoff gebundenen Acylgruppen, die mit aus einem Bleichmittel freigesetztem Wasserstoffperoxid in der Waschflotte zu Peroxycarbonsäuren reagieren. Beispiele für solche Verbindungen sind mehrfach acylierte Alkylendiamine, wie insbesondere Tetraacetylethylendiamin (TAED); acylierte Triazinderivate, insbesondere 1,5-Diacetyl-2,4-dioxohexahydro-1,3,5-triazin (DADHT); acylierte Glykolurile, insbesondere Tetraacetylglykoluril (TAGU); N-Acylimide, insbesondere N-Nonanoylsuccinimid (NOSI); acylierte Phenolsulfonate, insbesondere n-Nonanoyl- oder iso-Nonanoyloxybenzolsulfonat (n- bzw. iso-NOBS); Carbonsäureanhydride, wie Phthalsäureanhydrid; acylierte mehrwertige Alkohole, wie Ethylenglykoldiacetat, 2,5-Diacetoxy-2,5-dihydrofuran, acetyliertes Sorbitol und Mannitol und acylierte Zucker, wie Pentaacetylglucose; Enolester; sowie N-acylierte Lactame, insbesondere N-Acylcaprolactame und N-Acylvalerolactame. Ebenfalls als Bleichaktivatoren geeignet sind aminofunktionalisierte Nitrile und deren Salze (Nitrilquats), die zum Beispiel aus der Zeitschrift Tenside Surf. Det. 1997, 34(6), Seiten 404-409 bekannt sind. Als Bleichaktivatoren können außerdem Übergangsmetallkomplexe eingesetzt werden, die Wasserstoffperoxid zur bleichenden Fleckentfernung aktivieren können. Geeignete Übergangsmetallkomplexe sind beispielsweise bekannt aus EP-A 0 544 490 Seite 2, Zeile 4 bis Seite 3, Zeile 57; WO 00/52124 Seite 5, Zeile 9 bis Seite 8, Zeile 7 und Seite 8, Zeile 19 bis Seite 11, Zeile 14; WO 04/039932, Seite 2, Zeile 25 bis Seite 10, Zeile 21; WO 00/12808 Seite 6, Zeile 29 bis Seite 33, Zeile 29; WO 00/60043 Seite 6, Zeile 9 bis Seite 17, Zeile 22; WO 00/27975, Seite 2, Zeilen 1 bis 18 und Seite 3, Zeile 7 bis Seite 4, Zeile 6; WO 01/05925, Seite 1, Zeile 28 bis Seite 3, Zeile 14; WO 99/64156, Seite 2, Zeile 25 bis Seite 9, Zeile 18; sowie GB-A 2 309 976, Seite 3, Zeile 1 bis Seite 8, Zeile 32.

Die erfindungsgemäßen Waschmittel können weiterhin Enzyme enthalten, die die Reinigungswirkung verstärken, insbesondere Lipasen, Cutinasen, Amylasen, neutrale und alkalische Proteasen, Esterasen, Zellulasen, Pektinasen, Lactasen und/oder Peroxidasen. Die Enzyme können dabei an Trägerstoffe adsorbiert oder in Hüllsubstanzen eingebettet sein, um sie gegen Zersetzung zu schützen.

Die erfindungsgemäßen Waschmittel können außerdem chelatisierende Komplexbildner für Übergangsmetalle enthalten, mit denen sich eine katalytische Zersetzung von Aktivsauerstoffverbindungen in der Waschflotte vermeiden lässt. Geeignet sind zum Beispiel Phosphonate, wie Hydroxyethan-1,1-disphosphonat, Nitrilotrimethylenphosphonat, Diethylentriamin-penta(methylenphosphonat), Ethlyendiamin-tetra(methylenphosphonat), Hexamethylendiamin-tetra(methylenphosphonat) und deren Alkalimetallsalze. Ebenfalls geeignet sind Nitrilotriessigsäure und Polyaminocarbonsäuren, wie insbesondere Ethylendiamintetraessigsäure, Diethylentriaminpentaessigsäure, Ethylendiamin-N,N'-disuccinsäure, Methylglycindiessigsäure und Polyaspartate, sowie deren Alkalimetall- und Ammoniumsalze. Schließlich sind auch mehrwertige Carbonsäuren und insbesondere Hydroxycarbonsäuren, wie insbesondere Weinsäure und Zitronensäure als chelatisierende Komplexbildner geeignet.

Die erfindungsgemäßen Waschmittel können zusätzlich Vergrauungsinhibitoren enthalten, die von der Faser abgelösten Schmutz suspendiert halten und ein Wiederaufziehen des Schmutzes auf die Faser verhindern. Geeignete Vergrauungsinhibitoren sind zum Beispiel Celluloseether, wie Carboxymethylcellulose und deren Alkalimetallsalze, Methylcellulose, Hydroxyethylcellulose und Hydroxypropylcellulose. Ebenfalls geeignet ist Polyvinylpyrrolidon.

Die erfindungsgemäßen Waschmittel können weiterhin auch Schauminhibitoren enthalten, die die Schaumbildung in der Waschflotte verringern. Geeignete Schauminhibitoren sind zum Beispiel Organopolysiloxane wie Polydimethylsiloxan, Paraffine und/oder Wachse, sowie deren Gemische mit feinteiligen Kieselsäuren.

Die erfindungsgemäßen Waschmittel können gegebenenfalls optische Aufheller enthalten, die auf die Faser aufziehen, im UV-Bereich Licht absorbieren und blau fluoreszieren, um eine Vergilbung der Faser auszugleichen. Geeignete optische Aufheller sind zum Beispiel Derivate der Diaminostilbendisulfonsäure, wie Alkalimetallsalze von 4,4'-Bis-(2-anilino-4-morpholino-1,3,5-triazinyl-6-amino)-stilben-2,2'-disulfonsäure oder substituierte Diphenylstyryle, wie Alkalimetallsalze von 4,4'-Bis-(2-sulfostyryl)-diphenyl.

Die erfindungsgemäßen Waschmittel können schließlich auch noch Farbstoffe, sowie zusätzlich zu den erfindungsgemäßen Organopolysiloxanen weitere Duftstoffe enthalten.

Erfindungsgemäße Waschmittel in flüssiger Form oder Gelform können zusätzlich noch bis zu 30 Gew.-% organische Lösungsmittel, wie zum Beispiel Methanol, Ethanol, n-Propanol, Isopropanol, n-Butanol, Ethylenglykol, 1,2-Propylenglykol, 1,3-Propylenglykol, 1,4-Butylenglykol, Glycerin, Diethylenglykol, Ethylenglykolmethylether, Ethanolamin, Diethanolamin und/oder Triethanolamin enthalten.

Die erfindungsgemäßen Körperpflegemittel enthalten mindestens ein erfindungsgemäßes Organopolysiloxan und sind für die Pflege und/oder Reinigung von menschlicher Haut und/oder Haaren vorgesehen. Der Begriff Körperpflegemittel im Sinne der Erfindung umfasst dabei unter anderem Seifen, Flüssigseifen, Duschgele, Reinigungs- und Pflegelotionen, Hautcremes, Shampoos, Haarspülungen und Sonnenschutzmittel.

Die erfindungsgemäßen Körperpflegemittel können alle nach dem Stand der Technik bekannten Bestandteile von Körperpflegemitteln enthalten, wobei das Körperpflegemittel vorzugsweise keine sauer reagierenden Komponenten in nicht neutralisierter Form enthält. Die erfindungsgemäßen Körperpflegemittel können neben mindestens einem erfindungsgemäßen Organopolysiloxan als weitere Komponenten beispielsweise noch Tenside, Emulgatoren, Ölkörper, Fette und Wachse, Perlglanzwachse, Überfettungsmittel, Filmbildner, Hydrotrope, Viskositätsregulatoren, Konservierungsmittel, Feuchthaltemittel, Silikone, Antioxidantien, UV-Lichtschutzfilter, Antischuppenwirkstoffe, deodorierende Wirkstoffe, biogene Wirkstoffe, Farbstoffe, sowie Parfümöle enthalten.

Die erfindungsgemäßen Körperpflegemittel können Tenside enthalten, insbesondere wenn sie zur Reinigung von Haut und/oder Haaren bestimmt sind. Vorzugsweise werden dabei milde Tenside eingesetzt, die wenig hautreizend wirken. Dabei können sowohl anionische, als auch nichtionische, kationische und/oder amphotere Tenside verwendet werden.

Für die erfindungsgemäßen Körperpflegemittel geeignete anionische Tenside sind Seifen, Alkylbenzolsulfonate, Alkansulfonate, Olefinsulfonate, Alkylethersulfonate, Glycerinethersulfonate, alpha-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sufotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate.

Für die erfindungsgemäßen Körperpflegemittel geeignete nichtionische Tenside sind Fettalkoholpolyglykolether, Alkylphenolpolyglykolether, Fettsäurepolyglykolester, Fettsäureamidpolyglykolether, Fettaminpolyglykolether, alkoxylierte Triglyceride, partiell oxidierte Alk(en)yloligoglycoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide.

Für die erfindungsgemäßen Körperpflegemittel geeignete kationische Tenside sind quartäre Ammoniumverbindungen, wie beispielsweise Dimethyldistearylammoniumchlorid, Imidazoliniumquats und Esterquats, insbesondere quaternierte Fettsäuretrialkanolaminestersalze.

Für die erfindungsgemäßen Körperpflegemittel geeignete amphotere Tenside sind Alkylbetaine, Alkylamidobetaine, Aminopropionate, Aminoglycinate und Sulfobetaine.

Für die erfindungsgemäßen Körperpflegemittel bevorzugte milde Tenside sind Fettalkoholpolyglykolethersulfate, Monoglyceridsulfate, Monoalkylsulfosuccinate, Dialkylsulfosuccinate, Fettsäureisethionate, Fettsäuresarcosinate, Fettsäuretauride, Fettsäureglutamate, alpha-Olefinsulfonate, Ethercarbonsäuren, Alkyloligoglucoside, Fettsäureglucamide, Alkylamidobetaine, und Proteinfettsäurekondensate.

Die erfindungsgemäßen Körperpflegemittel können Emulgatoren enthalten, mit denen aus Wasser und wasserunlöslichen Bestandteilen stabile Emulsionen vom O/W-Typ oder W/O-Typ erhalten werden. Der Begriff Emulsionen umfasst dabei sowohl flüssige Zubereitungen, als auch cremeförmige und halbfeste Zubereitungen. Für die erfindungsgemäßen Körperpflegemittel können sowohl anionische, als auch nichtionische, kationische und/oder amphotere Emulgatoren verwendet werden.

Für die erfindungsgemäßen Körperpflegemittel geeignete anionische Emulgatoren sind aliphatische Fettsäuren mit 12 bis 22 Kohlenstoffatomen, wie beispielsweise Palmitinsäure, Stearinsäure oder Behensäure sowie Dicarbonsäuren mit 12 bis 22 Kohlenstoffatomen, wie beispielsweise Azelainsäure oder Sebacinsäure.

Für die erfindungsgemäßen Körperpflegemittel geeignete nichtionische Emulgatoren sind Alkoxylierungsprodukte von linearen Fettalkoholen mit 8 bis 22 Kohlenstoffatomen, Fettsäuren mit 12 bis 22 Kohlenstoffatomen, Alkylphenolen mit 14 bis 21 Kohlenstoffatomen, Alkylaminen mit 8 bis 22 Kohlenstoffatomen sowie von Rizinusöl und/oder gehärtetem Rizinusöl, wobei die Alkoxylierungsprodukte vorzugsweise 2 bis 30 Mol Ethylenoxid und/oder 1 bis 5 Mol Propylenoxid enthalten. Ebenfalls geeignet sind Alkyloligoglycoside mit 8 bis 18 Kohlenstoffatomen im Alkylrest; Partialglyceride, wie Hydroxystearinsäuremonoglycerid, Hydroxystearinsäurediglycerid, Isostearinsäuremonoglycerid, Isostearinsäurediglycerid, Ölsäuremonoglycerid Ölsäurediglycerid, Ricinolsäuremonoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Zitronensäuremonoglycerid, Zitronensäurediglycerid, Äpfelsäuremonoglycerid oder Äpfelsäurediglycerid; Sorbitanester, wie Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat oder Sorbitantrimaleat; sowie die Ethoxylierungsprodukte dieser Verbindungen mit 2 bis 30 Mol Ethylenoxid. Geeignete nichtionische Emulgatoren sind außerdem Polyglycerinester, wie Polyglyceryl-2 Dipolyhydroxystearat (Dehymuls PGPH), Polyglycerin-3 Diisostearat (Lameform TGI), Polyglyceryl-4 Isostearat (Isolan GI 34), Polyglyceryl-3 Oleat, Diisostearoyl Polyglyceryl-3 Diisostearat (Isolan PDI), Polyglyceryl-3 Methylglucose Distearat (Tego Care 450), Polyglyceryl-3 Beeswax (Cera Bellina), Polyglyceryl-4 Caprat (Polyglycerol Caprate T2010/90), Polyglyceryl-3 Cetylether (Chimexane NL), Polyglyceryl-3 Distearat (Cremophor GS32) und Polyglyceryl Polyricinoleat (Admul WOL 1403).

Für die erfindungsgemäßen Körperpflegemittel geeignete kationische Emulgatoren sind quaternierte Fettsäurealkanolaminester, die durch Veresterung von Alkanolaminen, vorzugsweise Triethanolamin oder Methyldiethanolamin, mit Fettsäuren und anschließende Quaternierung mit einem Alkylierungsmittel, vorzugsweise Dimethylsulfat oder Ethylenoxid, erhalten werden. Als Fettsäuren werden vorzugsweise Fettsäuregemische natürlicher Herkunft verwendet, die aus pflanzlichen oder tierischen Ölen oder Fetten gewonnen wurden und überwiegend C16- und C18-Fettsäuren enthalten, wobei die Fettsäuregemische mit dem natürlichen Unsättigungsgrad, partiell hydriert oder vollständig hydriert eingesetzt werden können. Vorzugsweise werden Fettsäuregemische mit einer Iodzahl im Bereich von 5 bis 50 verwendet. Besonders bevorzugt sind quaternierte Fettsäuretriethanolaminester mit einem mittleren Veresterungsgrad im Bereich von 1,5 bis 2,0 Mol Fettsäure pro Mol Triethanolamin.

Für die erfindungsgemäßen Körperpflegemittel geeignete amphotere Emulgatoren sind Betaine, wie N-Alkyl-N,N-dimethylammoniumglycinate, N-Acylaminopropyl-N,N-dimethylammoniumglycinate und 2-Alkyl-3-carboxymethyl-3-hydroxyethylimidazoline mit jeweils 8 bis 18 Kohlenstoffatomen in der Alkyl- oder Acylgruppe, vorzugsweise die unter der CTFA-Bezeichnung Cocoamidopropylbetain bekannte Verbindung. Ebenfalls geeignet sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 Kohlenstoffatomen in der Alkylgruppe.

Die erfindungsgemäßen Körperpflegemittel können Ölkörper enthalten, die wasserunlösliche Wirkstoffe lösen können und allein oder in Kombination mit Emulgatoren die sensorischen Eigenschaften der Körperpflegemittel bestimmen. Ölkörper im Sinn der Erfindung sind bei 20°C flüssige, mit Wasser bei 25°C nicht mischbare Stoffe oder Gemische von Stoffen.

Als Ölkörper eignen sich Guerbetalkohole auf Basis von Fettalkoholen mit 6 bis 18, vorzugsweise 8 bis 10 Kohlenstoffatomen (zum Beispiel Eutanol G); Ester von linearen C₆-C₂₂-Fettsäuren mit linearen oder verzweigten C₆-C₂₂-Fettalkoholen und Ester von verzweigten C₆-C₁₃-Carbonsäuren mit linearen oder verzweigten C₆-C₂₂-Fettalkoholen, wie zum Beispiel Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat; Ester von C₃-C₃₈-Alkylhydroxycarbonsäuren mit linearen oder verzweigten C₆-C₂₂-Fettalkoholen, zum Beispiel Diethylhexylmalat; sowie Ester von linearen oder verzweigten Fettsäuren mit mehrwertigen Alkoholen, wie zum Beispiel Propylenglykol, Dipropylenglykol oder Tripropylenglykol. Ebenfalls geeignet sind Triglyceride auf Basis C₆-C₁₀-Fettsäuren; flüssige Mono-, Di- und Triglyceridmischungen auf Basis von C₆-C₁₈-Fettsäuren; Ester von C₆-C₂₂-Fettalkoholen oder Guerbetalkoholen mit aromatischen Carbonsäuren, wie Benzoesäure; Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen; pflanzliche Öle; verzweigte primäre Alkohole; lineare und verzweigte C₆-C₂₂-Fettalkoholcarbonate, wie zum Beispiel Dicaprylylcarbonat (Cetiol CC); Guerbetcarbonate auf Basis von Fettalkoholen mit 6 bis 18, vorzugsweise 8 bis 10 Kohlenstoffatomen; Ester der Benzoesäure mit linearen oder verzweigten C₆-C₂₂-Alkoholen, zum Beispiel Finsolv TN; lineare oder verzweigte, symmetrische oder unsymmetrische Dialkylether mit 6 bis 22 Kohlenstoffatomen pro Alkylgruppe, wie zum Beispiel Dicaprylylether (Cetiol OE); Ringöffnungsprodukte von epoxidierten Fettsäureestern mit Polyolen, zum Beispiel Hydagen HSP, Sovermol 750, Sovermol 1102; sowie aliphatische Kohlenwasserstoffe, wie zum Beispiel Mineralöl, Vaseline, Petrolatum, Squalan, Squalen oder Dialkylcyclohexane.

Die erfindungsgemäßen Körperpflegemittel können Fette und Wachse enthalten, mit denen sich die sensorischen Eigenschaften insbesondere von cremeförmigen und halbfesten Körperpflegemittel einstellen lassen. Als Wachse eignen sich natürliche Wachse, wie zum Beispiel Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum und Paraffinwachse; chemisch modifizierte, gehärtete Wachse, wie zum Beispiel Montanesterwachse, Sasolwachse und hydrierte Jojobawachse; sowie synthetische Wachse, wie zum Beispiel Polyalkylenwachse und Polyethylenglykolwachse. Als Fette eignen sich tierische und pflanzliche Fette, die im wesentlichen aus gemischten Glycerinestern höherer Fettsäuren bestehen. An Stelle von Fetten können auch Phospholipide, Phosphatidylcholine, Sphingosine und Sphingolipide verwendet werden.

Die erfindungsgemäßen Körperpflegemittel können weiterhin Perlglanzwachse enthalten, die dem Körperpflegemittel durch einen perlmuttartigen Glanz ein attraktives Aussehen verleihen. Als Perlglanzwachse eignen sich Alkylenglykolester, wie Ethylenglykoldistearat; Fettsäurealkanolamide, wie Kokosfettsäurediethanolamid; Partialglyceride, wie Stearinsäuremonoglycerid; Ester von mehrwertigen Carbonsäuren, die mit Hydroxylgruppen substituiert sein können, mit Fettalkoholen mit 6 bis 22 Kohlenstoffatomen, insbesondere langkettige Ester der Weinsäure; Fettalkohole, Fettalkoholether und Fettalkoholcarbonate, die in Summe mindestens 24 Kohlenstoffatome aufweisen, wie Distearylether; Fettsäuren, wie Stearinsäure, Hydroxystearinsäure oder Behensäure; Ringöffnungsprodukte von Olefinoxiden mit 12 bis 22 Kohlenstoffatomen mit Fettalkoholen mit 12 bis 22 Kohlenstoffatomen oder Polyolen mit 2 bis 15 Kohlenstoffatomen und 2 bis 10 Hydroxylgruppen.

Die erfindungsgemäßen Körperpflegemittel können außerdem Überfettungsmittel enthalten, wie Lanolin oder Lecithin oder Ethoxylate dieser Verbindungen, Polyolfettsäureester, Monoglyceride oder Fettsäurealkanolamide.

Die erfindungsgemäßen Körperpflegemittel können weiterhin Filmbildner enthalten, mit denen sich Wirkstoffe auf Haut oder Haaren fixieren lassen. Als Filmbildner eignen sich Chitosan, mikrokristallines Chitosan, quaterniertes Chitosan, Polyvinylpyrrolidon, Vinylpyrrolidon-VinylacetatCopolymere, Polymere auf Basis von Acrylsäure und Methacrylsäure, quaternierte Cellulosen, Kollagen, Hyaluronsäure und deren Salze.

Zur Verbesserung des Fließverhaltens können die erfindungsgemäßen Körperpflegemittel außerdem Hydrotrope enthalten, wie Ethanol, Isopropanol oder Polyole. Besonders geeignet sind Polyole mit 2 bis 15 Kohlenstoffatomen und mindestens zwei Hydroxylgruppen, die optional weitere funktionelle Gruppen, insbesondere Aminogruppen, enthalten können. Beispiele für solche Polyole sind Glycerin; Alkylenglykole, wie Ethylenglykol, Diethylenglykol, Propylenglykol, Butylenglykol, Pentylenglykol, Hexylenglykol, sowie Polyethylenglykole mit einem durchschnittlichen Molekulargewicht von 100 bis 1.000 g/mol; Oligoglyceringemische mit einem Kondensationsgrad von 1,5 bis 10, wie technische Diglyceringemische mit einem Diglyceringehalt von 40 bis 50 Gew.-%; Methylolverbindungen, wie Trimethylolethan, Trimethylolpropan, Trimethylolbutan, Pentaerythrit und Dipentaerythrit; kurzkettige Alkyglucoside mit 1 bis 8 Kohlenstoffen im Alkylrest, wie Methyl- und Butylglucoside; Zuckeralkohole mit 5 bis 12 Kohlenstoffatomen, wie Sorbit oder Mannit; Zucker mit 5 bis 12 Kohlenstoffatomen, wie Glucose oder Saccharose; Aminozucker, wie Glucamin; sowie Dialkanolamine, wie Diethanolamin oder 2-Amino-1,3-propandiol.

Die erfindungsgemäßen Körperpflegemittel können zusätzlich Viskositätsregulatoren enthalten, um die Viskosität des Mittels zu erhöhen und gegebenenfalls das Mittel zu verdicken. Als Viskositätsregulatoren eignen sich natürliche Hydrogelbildner, wie Xanthan-Gum, Guar, Agar-Agar, Alginate und Tylosen; modifizierte Polysaccacharide, wie Celluloseether und Celluloseester, zum Beispiel Carboxymethylcellulose, Methylcellulose, Hydroxyethylcellulose, Methylhydroxyethylcellulose und Methylhydroxypropylcellulose; anorganische Hydrokolloide, wie Bentonite, Magnesium-Aluminium-Silkate und Siliciumdioxid; synthetische Hydrokolloide, wie Polyacrylate (im Handel erhältlich als Carbopole und Pemulen-Typen von Goodrich, Synthalene von Sigma, Keltrol-Typen von Kelco, Sepigel-Typen von Seppic und Salcare-Typen von Allied Colloids), unvernetzte und mit Polyolen vernetzte Polyacrylsäuren, Polyacrylamide, Polyvinylalkohole und Polyvinylpyrrolidone; Elektrolyte, wie Kochsalz und Ammoniumchlorid; sowie anionische, zwitterionische, amphotere und nichtionische Copolymere, wie Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, Acrylamidopropyltrimethylammoniumchlorid/Acrylat-Copolymere, Octylacrylamid/Methylmethacrylat/tert-Butylaminoethylmethacrylat/2-Hydroxypropylmethacrylat-Copolymere, Vinylpyrrolidon/Vinylacetat-Copolymere und Vinylpyrrolidon/Dimethylaminoethylmethacrylat/Vinylcaprolactam-Terpolymere. Weitere geeignete Verdickungsmittel sind in Cosm. Toil. 108, 95 (1993) aufgeführt.

Die erfindungsgemäßen Körperpflegemittel können außerdem Konservierungsmittel enthalten, mit denen sich vermeiden lässt, dass Inhaltsstoffe des Körperpflegemittels während dessen Aufbewahrung durch Bakterien abgebaut werden. Als Konservierungsmittel eignen sich Phenoxyethanol, Formaldehydlösung, Parabene, Pentandiol oder Sorbinsäure sowie die unter der Bezeichnung Surfacine TM bekannten Silberkomplexe. Weitere geeignete Konservierungsmittel sind aus Anlage 6, Teil A und B der deutschen Kosmetikverordnung bekannt.

Die erfindungsgemäßen Körperpflegemittel können zusätzlich Feuchthaltemittel enthalten, die eine Feuchtigkeitsregulierung der behandelten Haut bewirken und zusätzlich die sensorischen Eigenschaften des Körperpflegemittels verbessern können. Als Feuchthaltemittel eignen sich natürliche Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate. Ebenfalls geeignet sind Polyole und Polyolderivate, wie Glycerin, Diglycerin, Triglycerin, Ethylenglykol, Propylenglykol, Butylenglykol, Pentylenglykol, Erythrit und 1,2,6-Hexantriol; Polyethylenglykole, wie PEG-4, PEG-6, PEG-7, PEG-8, PEG-9, PEG-10, PEG-12, PEG-14, PEG-16, PEG- 18 und PEG-20; Zucker und Zuckerderivate, wie Fructose, Glucose, Maltose, Maltitol, Mannit, Inosit, Sorbit, Sucrose, Trehalose, Xylose, Xylit und Glucuronsäure und deren Salze; sowie ethoxyliertes Sorbit, wie Sorbeth-6, Sorbeth-20, Sorbeth-30 und Sorbeth-40. Geeignet sind außerdem Honig und gehärteter Honig, gehärtete Stärkehydrolysate, sowie Mischungen aus gehärtetem Weizenprotein und PEG-20-Acetatcopolymer. Vorzugsweise werden als Feuchthaltemittel Glycerin, Diglycerin, Triglycerin oder Butylenglykol eingesetzt.

Die erfindungsgemäßen Körperpflegemittel können weiterhin Silikone enthalten vom Strukturtyp der Cyclomethicone, Dimethicone, Dimethylpolysiloxane, Methylphenylpolysiloxane, cyclischen Silicone, sowie amino-, fettsäure-, alkohol-, polyether-, epoxy-, fluor-, glykosid- und alkylmodifizierten Siliconverbindungen. Geeignet sind außerdem Simethicone, bei denen es sich um Mischungen aus Dimethiconen mit einer durchschnittlichen Kettenlänge von 200 bis 300 Dimethylsiloxan-Einheiten und Siliziumdioxid oder hydrierten Silicaten handelt.

Die erfindungsgemäßen Körperpflegemittel können zusätzlich Antioxidantien enthalten, die die photochemische Reaktionskette unterbrechen können, welche ausgelöst wird, wenn UV-Strahlung in die Haut eindringt. Als Antioxidantien eignen sich Aminosäuren, wie Glycin, Histidin, Tyrosin und Tryptophan und deren Derivate; Peptide, wie D,L-Carnosin, D-Carnosin und L-Carnosin und deren Derivate, wie zum Beispiel Anserin; Carotinoide und, Carotine, wie alpha-Carotin, beta-Carotin und Lycopin und deren Derivate; Chlorogensäure und deren Derivate; Liponsäure und deren Derivate, wie Dihydroliponsäure; Aurothioglucose, Propylthiouracil und andere Thiole, wie Thioredoxin, Glutathion, Cystein, Cystin und Cystamin und deren Glycosyl-, N-Acetyl-, Methyl-, Ethyl-, Propyl-, Amyl-, Butyl-, Lauryl-, Palmitoyl-, Oleyl-, gamma-Linoleyl-, Cholesteryl- und Glycerylester, sowie deren Salze; Dilaurylthiodipropionat, Distearylthiodipropionat und Thiodipropionsäure und deren Ester, Ether, Peptide, Lipide, Nukleotide, Nukleoside und Salze; Sulfoximinverbindungen, wie Butioninsulfoximine, Homocysteinsulfoximin, Butioninsulfone und Penta-, Hexa- und Heptathioninsulfoximin; Metall-chelatisierende Verbindungen, wie alpha-Hydroxyfettsäuren, Palmitinsäure, Phytinsäure, Lactoferrin, alpha-Hydroxysäuren, insbesondere Citronensäure, Milchsäure und Äpfelsäure, Huminsäure, Gallensäure, Gallenextrakte, Bilirubin, Biliverdin, EDTA und EGTA; ungesättigte Fettsäuren und deren Derivate, wie gamma-Linolensäure, Linolsäure und Ölsäure; Folsäure und deren Derivate; Ubichinon und Ubichinol und deren Derivate; Vitamin C und dessen Derivate, wie Ascorbylpalmitat, Mg-Ascorbylphosphat und Ascorbylacetat; Tocopherole und deren Derivate, wie Vitamin-E-acetat; sowie Vitamin A und dessen Derivate, wie Vitamin-A-palmitat. Als Antioxidantien geeignet sind außerdem Rutinsäure und deren Derivate, wie alpha-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate, wie Zinkoxid, Selen und dessen Derivate, wie Selenomethionin), sowie Stilbene und deren Derivate, wie Stilbenoxid.

Die erfindungsgemäßen Körperpflegemittel können außerdem UV-Lichtschutzfilter enthalten, die UV-A-Strahlung und/oder UV-B-Strahlung absorbieren. Als UV-Lichtschutzfilter können dabei sowohl organische Substanzen, als auch anorganische Pigmente eingesetzt werden, wobei die organischen Substanzen sowohl öllöslich oder wasserlöslich, als auch unlöslich sein können. Geeignete öllösliche UV-B-Lichtschutzfilter sind 3-Benzylidencampher und 3-Benzylidennorcampher und deren Derivate, wie 3-(4-Methylbenzyliden)campher, bekannt aus EP-A 0 693 471; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, und 2-Cyano-3,3-phenylzimtsäure-2-ethylhexylester (Octocrylene); Ester der Salicylsäure, vorzugsweise Salicylsäure-2-ethylhexylester, Salicylsäure-4-iso-propylbenzylester und Salicylsäurehomomenthylester; Derivate des Benzophenons, vorzugsweise 2-Hydroxy-4-methoxybenzophenon, 2-Hydroxy-4-methoxy-4'-methylbenzophenon und 2,2'-Dihydroxy-4-methoxybenzophenon; Ester der Benzalmalonsäure, vorzugsweise 4-Methoxybenzmalonsäuredi-2-ethylhexyl-ester; Triazinderivate, wie 2,4,6-Trianilino-(p-carbo-2'-ethyl-1'-hexyloxy)-1,3,5-triazin, Octyltriazon, bekannt aus EP-A 0 818 450 und Dioctylbutamidotriazon (Uvasorb HEB); Propan-1,3-dione, wie 1-(4-tert.Butylphenyl)-3-(4'methoxyphenyl)propan-1,3-dion; sowie Ketotricyclo(5.2.1.0)decan-Derivate, bekannt aus EP-A 0 694 521. Geeignete wasserlösliche UV-B-Lichtschutzfilter sind 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und deren Salze; sowie Sulfonsäurederivate des 3-Benzylidencamphers, wie 4-(2-Oxo-3-bornylidenmethyl)benzolsulfonsäure und 2-Methyl-5-(2-oxo-3-bornyliden)sulfonsäure und deren Salze. Als organische UV-A-Lichtschutzfilter eignen sich Derivate des Benzoylmethans, wie 1-(4'-tert-Butylphenyl)-3-(4'-methoxyphenyl)propan-1,3-dion, 4-tert-Butyl-4'-methoxydibenzoylmethan (Parsol 1789) und 1-Phenyl-3-(4'-isopropylphenyl)-propan-1,3-dion, sowie die aus DE 191 12 033 bekannten Enaminverbindungen. Die UV-A- und UV-B-Lichtschutzfilter können selbstverständlich auch in Mischungen eingesetzt werden. Bevorzugte Kombinationen bestehen aus Derivaten des Benzoylmethans, wie 4-tert-Butyl-4'-methoxydibenzoylmethan, und 2-Cyano-3,3-phenylzimtsäure-2-ethyl-hexylester in Kombination mit Estern der Zimtsäure, wie 4-Methoxyzimtsäure-2-ethylhexylester, 4-Methoxyzimtsäurepropylester oder 4-Methoxyzimtsäureisoamylester. Vorteilhaft werden derartige Kombinationen mit wasserlöslichen Filtern, wie 2-Phenylbenzimidazol-5-sulfonsäure und deren Alkali-, Erdalkali-, Ammonium-, Alkylammonium-, Alkanolammonium- und Glucammoniumsalze kombiniert. Als anorganische Lichtschutzpigmente eignen sich feindisperse Metalloxidpulver, die bevorzugt in hydrophobierter Form vorliegen, insbesondere Titandioxidpulver, Aluminiumoxidpulver, Zinkoxidpulver und Mischoxidpulver mit den Elementen Si, Ti, Al, Zn, Fe, B, Zr, und/oder Ce. Vorzugsweise werden sogenannte Mikro- oder Nanopigmente eingesetzt, deren Partikel einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Beispiele für handelsübliche anorganische Lichtschutzpigmente sind gecoatete Titandioxide UV-Titan M212, M 262 und X 111 der Firma Kemira, AEROXIDE TiO₂ P25, PF2, T 805 und T 817 der Firma Degussa, Micro Titanium Dioxide MT-150 W, MT-100 AQ, MT-100 SA, MT-100 HD und MT-100 TV der Firma Tayca, Eusolex T2000 der Firma Merck, Zinc Oxide neutral H&R und Zinc Oxide NDM der Firma Haarmann & Reimer, sowie Z-Cote und Z-Cote HP1 der Firma BASF. Weitere geeignete UV-Lichtschutzfilter sind aus der Übersicht von P. Finkel in SÖFW-Journal 122, 543 (1996) sowie Parf. Kosm. 3, 11 (1999) bekannt.

Erfindungsgemäße Körperpflegemittel zur Behandlung von Haaren können außerdem Antischuppenwirkstoffe enthalten. Als Antischuppenwirkstoffe eignen sich 1-Hydroxy-4-methyl-6-(2,4,4-trimethylpentyl)-2-(1H)-pyridinonmonoethanolamin, Climbazol, Ketoconazol, Elubiol, Selendisulfid, kolloidaler Schwefel, Schwefelpolyethylenglykolsorbitanmonooleat, Schwefelrizinolpolyethoxylat, Schwefelteer-Destillate, Salicylsäure in Kombination mit Hexachlorophen, Undecylensäuremonoethanolamidsulfosuccinat-Na, Protein-Undecylensäurekondensate, Zinkpyrithion, Aluminiumpyrithion und Magnesiumpyrithion.

Die erfindungsgemäßen Körperpflegemittel können zusätzlich auch deodorierende Wirkstoffe enthalten. Als deodorierende Wirkstoffe eignen sich adstringierende Metallsalze, die eine antitranspirante Wirkung haben, keimhemmende Mittel, Enzyminhibitoren und Geruchsabsorber, die einzeln oder in Kombination verwendet werden können. Geeignete adstringierende Metallsalze mit antitranspiranter Wirkung sind Aluminiumchlorhydrate, Aluminium-ZirkoniumChlorohydrate, Aluminiumhydroxylactate sowie Zinksalze. Geeignete Handlesprodukte sind Locron der Firma Clariant und Rezal 36G der Firma Reheis. Als keimhemmende Mittel sind grundsätzlich alle gegen gram-positive Bakterien wirksamen Stoffe geeignet, wie zum Beispiel 4-Hydroxybenzoesäure und ihre Salze und Ester, N-(4-Chlorphenyl)-N'-(3,4-dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), sowie Salicylsäure-N-alkylamide, wie Salicylsäure-n-octylamid oder Salicylsäure-n-decylamid. Als Enzyminhibitoren eignen sich Esteraseinhibitoren, vorzugsweise Trialkylcitrate, wie Trimethylcitrat, Tripropylcitrat, Triisopropylcitrat, Tributylcitrat und insbesondere Triethylcitrat; Sterolsulfate und Sterolphosphate, wie Lanosterin-, Cholesterin-, Campesterin-, Stigmasterin- und Sitosterinsulfat bzw. - phosphat; Dicarbonsäuren und deren Ester, wie Glutarsäure, Glutarsäuremonoethylester, Glutarsäurediethylester, Adipinsäure, Adipinsäuremonoethylester, Adipinsäurediethylester, Malonsäure und Malonsäurediethylester; sowie Hydroxycarbonsäuren und deren Ester wie beispielsweise Zitronensäure, Apfelsäure, Weinsäure oder Weinsäurediethylester. Als Geruchsabsorber eignen sich Stoffe, die geruchsbildende Verbindungen aufnehmen und weitgehend festhalten können, indem sie den Partialdruck der einzelnen Komponenten senken. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie Extrakte von Labdanum, Styrax oder bestimmte Abietinsäurederivate.

Die erfindungsgemäßen Körperpflegemittel können außerdem biogene Wirkstoffe, wie Tocopherol, Tocopherolacetat, Tocopherolpalmitat, Ascorbinsäure, (Desoxy)Ribonucleinsäure und deren Fragmentierungsprodukte, beta-Glucane, Retinol, Bisabolol, Allantoin, Phytantriol, Panthenol, Panthotensäure, Fruchtsäuren, alpha-Hydroxysäuren, Aminosäuren, Ceramide, Pseudoceramide, essentielle Öle, sowie Vitaminkomplexe enthalten. Als biogene Wirkstoffe können auch Pflanzenextrakte, vor allem Extrakte aus Arnika, Birke, Kamille, Klettenwurzel, Bartflechte, Pappel, Brennessel, Prunus-Arten, Bambaranus und von Walnusschalen verwendet werden.

Die erfindungsgemäßen Körperpflegemittel können weiterhin Farbstoffe enthalten, wobei sowohl lösliche Farbstoffe als auch unlösliche Pigmente geeignet sind. Als lösliche Farbstoffe eignen sich pflanzliche und tierische Farbstoffe, wie Betanin, Bixin, Carmin, Carotin, Clorophyll oder Sepia, sowie synthetische organische Farbstoffe, wie Azo-, Antrachinon- oder Triphenylmethanfarbstoffe, die vorzugsweise wasserlöslich oder in Wasser dispergierbar sind. Als unlösliche Pigmente eignen sich natürliche anorganische Pigmente, wie Ocker, Umbra, roter Bolus, Terra di Siena oder Kreide, sowie synthetische anorganische Pigmente, wie Eisenoxide, Ultramarine, Titandioxid, Zinkoxid oder Glimmer-basierte Pigmente, insbesondere Perlglanzpigmente.

Die erfindungsgemäßen Körperpflegemittel können schließlich auch noch Parfümöle enthalten, um dem Körperpflegemittel einen ansprechenden Geruch zu vermitteln. Die verwendeten Parfümöle sind üblicherweise Mischungen aus Riechstoffen, wobei sowohl natürliche als auch synthetische Riechstoffe verwendet werden können. Als natürliche Riechstoffe eignen sich Extrakte von Blüten, Stängeln und Blättern, Früchten, Fruchtschalen, Wurzeln, Hölzern, Kräutern und Gräsern, Nadeln und Zweigen, sowie Harze und Balsame. Geeignete tierische Riechstoffe sind beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind zum Beispiel Benzylacetat, p-tert-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden zum Beispiel die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen zum Beispiel die Ionone und Methylcedrylketon, zu den Alkoholen Anethol, Citronellol, Eugenol, Isoeugenol, Geraniol, Linalool, Phenylethanol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, zum Beispiel Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethanol, alpha-Hexylzimtaldehyd, Geraniol, Benzylaceton, Cyclamenaldehyd, Linalool, Boisambren Forte, Ambroxan, Indol, Hedion, Sandelice, Citronenöl, Mandarinenöl, Orangenöl, Allylamylglycolat, Cyclovertal, Lavandinöl, Muskateller Salbeiöl, beta-Damascon, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen eingesetzt.

Eine weitere bevorzugte Ausführungsform der erfindungsgemäßen Textilbehandlungsmittel sind Duftspüler. Duftspüler im Sinne der Erfindung sind flüssige Zubereitungen, die ein erfindungsgemäßes Organopolysiloxan in Form einer wässrigen Dispersion enthalten und aus denen das erfindungsgemäße Organopolysiloxan auf Textilfasern aufzieht, wenn der Duftspüler bei der Reinigung von Textilien in einer wässrigen Waschflotte in einem Spülvorgang, der nach dem eigentlichen Waschvorgang vorgenommen wird, dem zum Spülen verwendeten Wasser zugesetzt wird.

Duftspüler, die ein erfindungsgemäßes Organopolysiloxan in flüssiger Form enthalten, enthalten vorzugsweise noch mindestens einen Emulgator, der mit dem flüssigen Organopolysiloxan eine stabile O/W-Emulsion bildet. Als Emulgatoren für die erfindungsgemäßen Duftspüler eignen sich die zuvor als mögliche Bestandteile von erfindungsgemäßen Körperpflegemitteln genannten anionischen, nichtionischen und amphoteren Emulgatoren.

Die erfindungsgemäßen Duftspüler enthalten vorzugsweise auch noch puffernde Bestandteile, mit denen der pH-Wert des Duftspülers im Bereich von pH 5 bis 9, vorzugsweise pH 6 bis 8, gehalten wird.

Die erfindungsgemäßen Duftspüler können darüber hinaus auch noch weitere Komponenten wie Hydrotrope, Viskositätsregulatoren, Konservierungsmittel, Silikone und Parfümöle enthalten, wobei die bereits zuvor als mögliche Bestandteile von erfindungsgemäßen Körperpflegemitteln genannten Verbindungen geeignet sind. Außerdem können die erfindungsgemäßen Duftspüler auch Vergrauungsinhibitoren, Schauminhibitoren, optische Aufheller und Farbstoffe enthalten, die für die zuvor beschriebenen erfindungsgemäßen Waschmittel geeignet sind.

### Beispiele

### Beispiel 1

Herstellung von Chloressigsäurecitronellylester.

Eine Mischung aus 94,5 g (1,0 mol) Monochloressigsäure und 203,2 g (1,4 mol) 3,7-Dimethyl-6-octen-1-ol (Citronellol) wurde 6 h bei 50 mbar auf 130°C erhitzt, wobei das durch die Veresterung gebildete Wasser über eine Kolonne abdestilliert wurde. Die erhaltene Reaktionsmischung wies eine Säurezahl von 5 mg KOH/g auf. Nicht umgesetztes Citronellol wurde anschließend bei 6 mbar und einer Sumpftemperatur bis 130°C abdestilliert. Destillation des Rückstands bei 6 mbar lieferte 198 g Chloressigsäurecitronellylester als farblose Flüssigkeit.

### Beispiel 2

Herstellung eines Organopolysiloxans der Struktur

R⁶-O-[Si(CH₃)₂-O]ₙ-R⁶

mit n = 10, R⁶ = -CH₂-CH₂-N+(CH₃)₂-CH₂-C(O)OY Cl⁻ und YOH = Citronellol.

Eine Mischung aus 346 g (0,4 mol) alpha,omega-Bis-(2-dimethylaminoethoxy)-poly(dimethylsiloxan) mit einem mittleren Molekulargewicht von 865 g/mol und 163 g (0,7 mol) Chloressigsäurecitronellylester wurde für 11 h auf 60°C erhitzt. Das erhaltene Produkt wies einen Gehalt an freiem Chlorid von 4,43 Gew.-% und einen Gehalt an organisch gebundenem Chlorid von 0,05 Gew.-% auf, woraus sich ein Umsatz zum Betainester von 99% ergibt.

### Beispiel 3

Herstellung eines Organopolysiloxans der Struktur

R⁶-O-[Si(CH₃)₂-O]ₙ-R⁶

mit n = 50, R⁶ = -CH₂-CH₂-N⁺(CH₃)₂-CH₂-C(O)OY Cl⁻ und YOH = Citronellol.

Eine Mischung aus 308,5 g (0,08 mol) alpha,omega-Bis-(2-dimethylaminoethoxy)-poly(dimethylsiloxan) mit einem mittleren Molekulargewicht von 3856 g/mol und 32,6 g (0,14 mol) Chloressigsäurecitronellylester wurde für 56 h auf 60°C erhitzt. Das erhaltene Produkt wies einen Gehalt an freiem Chlorid von 2,29 Gew.-% und einen Gehalt an organisch gebundenem Chlorid von 0,04 Gew.-% auf, woraus sich ein Umsatz zum Betainester von 98% ergibt.

### Beispiel 4

pH-Abhängigkeit der Freisetzung von Citronellol aus wässrigen Dispersionen des Organopolysiloxans aus Beispiel 3.

Die Bestimmung der Freisetzung von Citronellol aus dem Organopolysiloxan erfolgte bei Zimmertemperatur in handelsüblichen Pufferlösungen mit pH-Werten von 3, 6 und 9. 0,5 g Organopolysiloxan aus Beispiel 3 und 0,5 g Emulgator Rewopal C6 (Cocosmonoethanolamidopolyglykolether) wurden mit Pufferlösung auf 50 ml aufgefüllt, im Ultraschallbad für 1 min emulgiert und die gebildeten stabilen Emulsionen wurden bei Zimmertemperatur gelagert. Nach den angegebenen Zeiten wurden Proben entnommen, mit Toluol extrahiert und der Anteil an freigesetztem Citronellol mit Gaschromatographie bestimmt. Die Tabelle 1 zeigt die Mengen an freigesetztem Citronellol in Abhängigkeit von der Zeit und dem pH-Wert der Pufferlösung, wobei die Zahlen jeweils Mittelwerte aus zwei Versuchen sind.

Die Messergebnisse zeigen, dass durch das Dispergieren mit Ultraschall bereits ca. 9 % des Citronellols freigesetzt wurden. Danach nahm bei pH 6 und pH 9 die Menge an freigesetztem Citronellol innerhalb von 24 h nur noch um ca. 2 % zu, d.h. der Betainester war stabil. Bei pH 3 wurden dagegen innerhalb von 24 h weitere ca. 42 % des Citronellols freigesetzt.

**Tabelle 1**

| Freigesetzte Menge an Citronellol | | | |
|---|---|---|---|
| Lagerdauer in h | pH 3 | pH 6 | pH 9 |
| 0,25 | 10,3 | 9,4 | 8,8 |
| 2 | | 9,8 | 8,7 |
| 2,5 | 17,3 | | |
| 24 | 52,0 | 11,1 | 10,8 |
| 36 | 62,0 | | 11,4 |

### Beispiel 5

Anwendung des Organopolysiloxans aus Beispiel 3 in einem Duftspüler, Aufziehen des Organopolysiloxans auf Textilfasern und Freisetzung von Citronellol durch Säureeinwirkung.

20 g des Organopolysiloxans aus Beispiel 3 und 5,0 g Rewopal LA6 (Laurylalkoholethoxylat mit 6 EO-Einheiten) wurden durch Rühren mit 75 g Pufferlösung von pH 7 emulgiert. Es wurden 100 g einer Duftspüler-Emulsion erhalten.

In einer Haushaltswaschmaschine wurden 3,5 kg Baumwoll-T-Shirts mit WFK Basiswaschmittel Parfumfrei bei 40°C gewaschen. Die Duftspüler-Emulsion wurde dem letzten Spülgang über die Einspülkammer der Waschmaschine zugegeben. Die gewaschenen T-Shirts wurden geschleudert und auf einer Leine an der Luft für mindestens 4 Tage getrocknet, bis kein Geruch mehr festzustellen war. Beim anschließenden Besprühen der T-Shirts mit einer 1 Gew.-% Zitronensäurelösung entwickelte sich ein deutlicher Geruch nach Citronellol. Auch nach vierwöchiger trockener Lagerung der T-Shirts wurde nach Besprühen mit der Zitronensäurelösung noch ein deutlicher Geruch nach Citronellol festgestellt.

### Beispiel 6

Anwendung des Organopolysiloxans aus Beispiel 3 in einem Duftspüler, weichmachende Wirkung des Organopolysiloxans auf Textilfasern.

3 kg Baumwollfrottiertücher 80*50 cm der Firma Frottana Textil mit 350 g/m² wurden zweimal mit je 75 g Waschmittel IEC-A Base der Firma WKF-Testgewebe und zweimal ohne Waschmittel in einem Waschvollautomaten Miele Mondia 1120 bei 95°C gewaschen und bei 1200 min⁻¹ geschleudert. Die gewaschenen Gewebe wurden auf einer Leine an der Luft getrocknet. 10 g einer wie in Beispiel 5 hergestellten Duftspüler-Emulsion wurden mit Leitungswasser unter Rühren auf 2 l verdünnt. Ein Stück des gewaschenen Testgewebes wurde einmal gefaltet in eine Wanne von 40*50 cm gelegt und mit der verdünnten Duftspüler-Emulsion übergossen. Das Gewebe wurde 10 min in der Wanne gehalten und dabei einmal umgewendet, anschließend mit 1200 min⁻¹ für 2 min geschleudert und auf einer Leine an der Luft getrocknet. Anschließend wurde der Weichgriff des behandelten Frottiertuchs von einem Panel mit 9 Testpersonen sensorisch bewertet, wobei Punkte von 0 (hart) bis 5 (sehr weich) vergeben wurden. Zum Vergleich wurde ein Frottiertuch beurteilt, das mit einer Duftspüler-Emulsion behandelt wurde, zu deren Herstellung an Stelle des Organopolysiloxans aus Beispiel 3 ein quaternäres Polysiloxan eingesetzt wurde, das die Struktur der Verbindung der Formel (III) aus EP-A 1 199 350 mit 50 an Stelle von 30 [Si(CH₃)₂O]-Einheiten aufwies. Ebenso wurde zum Vergleich ein Frottiertuch beurteilt, das mit Leitungswasser an Stelle der Duftspüler-Emulsion behandelt wurde.

**Tabelle 2**

| Weichgriff nach Behandlung mit Duftspüler-Emulsion | |
|---|---|
| Duftspüler-Wirkstoff | Weichgriff (max. 45 Punkte) |
| Organopolysiloxan aus Beispiel 3 | 32 |
| quaternäres Polysiloxan entsprechend EP-A 1 199 350* | 34 |
| ohne | 0 |

| | |
|---|---|
| *nicht erfindungsgemäß | |

Die Versuchsergebnisse zeigen, dass mit der erfindungsgemäßen Duftspüler-Emulsion bei Baumwollgewebe ein Weichgriff erzielt wird, der dem Weichgriff entspricht, der mit einem handelsüblichem Weichspüler erreicht wird.

## Patentansprüche

1. Organopolysiloxan, aus dem ein Riechstoffalkohol freigesetzt werden kann,
**dadurch gekennzeichnet,**
**dass** es mindestens eine funktionelle Gruppe Z der Struktur
-N⁺R¹R²-CH₂-C(O)OY A⁻
aufweist, worin
R¹, R² unabhängig voneinander ausgewählt sind aus C₁₋₃₀ Alkyl und Hydroxyethyl,
Y der Rest eines Riechstoffalkohols Y-OH ist und
A⁻ das Anion einer physiologisch verträglichen Säure HA ist.

2. Organopolysiloxan gemäß Anspruch 1,
**dadurch gekennzeichnet,**
**dass** es eine Struktur gemäß Formel (I)
(I) R³R⁴R⁵Si-O-CH₂-CH₂-Z
aufweist, worin
R³ ein über ein Sauerstoffatom gebundener Polysiloxanrest ist,
R⁴ eine C₁₋₃₀ Alkylgruppe oder Phenyl ist,
R⁵ = R³ oder R⁴ ist und
Z die gleiche Bedeutung wie in Anspruch 1 hat.

3. Organopolysiloxan gemäß Anspruch 1,
**dadurch gekennzeichnet,**
**dass** es eine Struktur gemäß Formel (I)
(I) R⁶-O-[Si(CH₃)₂-O]ₙ-R⁶
aufweist, worin
n = 3-200 ist,
R⁶ = -CH₂-CH₂-N⁺(CH₃)₂-CH₂-C(O)OY A⁻ ist und
Y und A⁻ die gleiche Bedeutung wie in Anspruch 1 haben.

4. Organopolysiloxan gemäß einem der Ansprüche 1 bis 3,
**dadurch gekennzeichnet,**
**dass** der Riechstoffalkohol Y-OH ausgewählt ist aus der Gruppe 4-Allyl-2-methoxyphenol (Eugenol), 3-(2-Bornyloxy)-2-methyl-1-propanol, 2-tert-Butylcyclohexanol, 4-tert-Butylcyclohexanol, Benzylalkohol, 1-Decanol, 9-Decen-1-ol, Dihydroterpineol, 2,4-Dimethyl-4-cyclohexen-1-ylmethanol, 2,4-Dimethylcyclohexylmethanol, 2,6-Dimethyl-2-heptanol, 2,6-Dimethyl-4-heptanol, 3a,4,5,6,7,7a-Hexahydro-2,4-dimethyl-4,7-methano[1H]inden-5-ol, 3,7-Dimethyl-1,6-nonadien-3-ol, 2,6-Dimethyl-2,7-octadien-6-ol (Linalool), cis-3,7-Dimethyl-2,6-octadien-1-ol (Nerol), trans-3,7-Dimethyl-2,6-octadien-1-ol (Geraniol), 3,7-Dimethyl-1,7-octandiol, 3,7-Dimethyl-1-octanol (Tetrahydrogeraniol), 2,6-Dimethyl-2-octanol (Tetrahydromyrcenol), 3,7-Dimethyl-3-octanol (Tetrahydrolinalool), 2,6-Dimethyl-7-octen-2-ol (Dihydromyrcenol), 3,7-Dimethyl-6-octen-1-ol (Citronellol), 2,2-Dimethyl-3-(3-methylphenyl)-1-propanol, 2,2-Dimethyl-3-phenyl-1-propanol, 2-Ethoxy-4-methoxymethylphenol, 2-Ethyl-4-(2,2,3-trimethyl-3-cyclopenten-1-yl)-2-buten-1-ol, cis-3-Hexen-1-ol, 4-(4-Hydroxy-3-methoxyphenyl)-2-butanon, 1-Hydroxy-2-(1-methyl-1-hydroxyethyl)-5-methylcyclohexan, 3-(Hydroxymethyl)-2-nonanon, 4-(4-Hydroxy-4-methylpentyl)-3-cyclohexen-1-carboxaldehyde, Isoborneol, 3-Isocamphylcyclohexanol, 2-Isopropenyl-5-methylcyclohexanol (Isopulegol), 1-Isopropyl-4-methylcyclohex-3-enol (Terpinenol), 4-Isopropylcyclohexanol, 1-(4-Isopropylcyclohexyl)-ethanol, 4-Isopropylcyclohexylmethanol, 2-Isopropyl-5-methylcyclohexanol (Menthol), 2-Isopropyl-5-methylphenol (Thymol), 5-Isopropyl-2-methylphenol (Carvacrol), 2-(4-Methyl-3-cyclohexenyl)-2-propanol (Terpineol), 2-(4-Methylcyclohexyl)-2-propanol (Dihydroterpineol), 4-Methoxybenzylalcohol, 2-Methoxy-4-methylphenol, 3-Methoxy-5-methylphenol, 1-Methoxy-4-propenylbenzol (Anethol), 2-Methoxy-4-propenylphenol (Isoeugenol), 4-Methyl-3-decen-5-ol, 2-Methyl-6-methylene-7-octen-2-ol (Myrcenol), 3-Methyl-4-phenyl-2-butanol, 2-(2-Methylphenyl)-ethanol, 2-Methyl-4-phenyl-1-pentanol, 3-Methyl-5-phenyl-1-pentanol, 2-Methyl-1-phenyl-2-propanol, (1-Methyl-2-(1,2,2-trimethylbicyclo[3.1.0]hex-3-ylmethyl)-cyclopropyl)-methanol, 3-Methyl-4-(2,2,6-trimethylcyclohexen-1-yl)-2-butanol, 2-Methyl-4-(2,2,3-trimethyl-3-cyclopenten-1-yl)-2-buten-1-ol, (3-methyl-1-(2,2,3-trimethyl-3-cyclopentenyl)-3-cyclohexen-1-yl)-methanol, 3-Methyl-5-(2,2,3-trimethyl-3-cyclopenten-1-yl)-4-penten-2-ol, 2-Methyl-2-vinyl-5-(1-hydroxy-1-methylethyl)-tetrahydrofuran, trans,cis-2,6-Nonadienol, 1-Nonanol, Nopol, 1,2,3,4,4a,5,6,7-Octahydro-2,5,5-trimethyl-2-naphthol, 1-Octanol, 3,4,5,6,6-Pentamethyl-2-heptanol, 2-Phenylethanol, 2-Phenylpropanol, 3-Phenylpropanol (Hydrozimtalkohol), 3-Phenyl-2-propen-1-ol (Zimtalkohol), 4-(5,5,6-Trimethylbicyclo[2.2.1]hept-2-yl)-cyclohexan-1-ol, 3,5,5-Trimethylcyclohexanol, 2,4,6-Trimethyl-4-cyclohexen-1-ylmethanol, 5-(2,2,3-Trimethyl-3-cyclopentenyl)-3-methylpentan-2-ol, 3,7,11-Trimethyl-2,6,10-dodecatrien-1-ol (Farnesol), 3,7,11-Trimethyl-1,6,10-dodecatrien-3-ol (Nerolidol), 3,5,5-Trimethyl-1-hexanol (Isononanol), 1-Undecanol, 10-Undecen-1-ol, Vetiverol.

5. Organopolysiloxan gemäß einem der Ansprüche 1 bis 4,
**dadurch gekennzeichnet,**
**dass** in einer wässrigen Dispersion des Organopolysiloxans der Riechstoffalkohol Y-OH bei einem pH-Wert von 3 schneller freigesetzt wird als bei einem pH-Wert von 9.

6. Textilbehandlungsmittel, enthaltend mindestens ein Organopolysiloxan gemäß einem der Ansprüche 1 bis 5.

7. Waschmittel, enthaltend mindestens ein Tensid und mindestens ein Organopolysiloxan gemäß einem der Ansprüche 1 bis 5.

8. Duftspüler, enthaltend mindestens ein Organopolysiloxan gemäß einem der Ansprüche 1 bis 5 in Form einer wässrigen Dispersion.

9. Körperpflegemittel, enthaltend mindestens ein Organopolysiloxan gemäß einem der Ansprüche 1 bis 5.

## Claims

1. An organopolysiloxane from which a fragrance alcohol can be released,
**characterised in that**
it comprises at least one functional group Z having the structure
-N⁺R¹R²-CH₂-C(O)OY A⁻
wherein
R¹, R² are mutually independently selected from C₁₋₃₀ alkyl and hydroxyethyl,
Y is the radical of a fragrance alcohol Y-OH and
A⁻ is the anion of a physiologically compatible acid HA.

2. An organopolysiloxane according to claim 1,
**characterised in that**
it has a structure according to formula (I)
(I) R³R⁴R⁵Si-O-CH₂-CH₂-Z
wherein
R³ is a polysiloxane radical bonded by an oxygen atom,
R⁴ is a C₁₋₃₀ alkyl group or phenyl,
R⁵ = R³ or R⁴ and
Z has the same meaning as in claim 1.

3. An organopolysiloxane according to claim 1,
**characterised in that**
it has a structure according to formula (I)
(I) R⁶-O-[Si(CH₃)₂-O]ₙ-R⁶
wherein
n = 3-200,
R⁶ = -CH₂-CH₂-N⁺(CH₃)₂-CH₂-C(O)OY A⁻ and
Y and A' have the same meaning as in claim 1.

4. An organopolysiloxane according to one of claims 1 to 3,
**characterised in that**
the fragrance alcohol Y-OH is selected from the group comprising 4-allyl-2-methoxyphenol (eugenol), 3-(2-bornyloxy)-2-methyl-1-propanol, 2-tert-butylcyclohexanol, 4-tert-butylcyclohexanol, benzyl alcohol, 1-decanol, 9-decen-1-ol, dihydroterpineol, 2,4-dimethyl-4-cyclohexen-1-yl methanol, 2,4-dimethylcyclohexyl methanol, 2,6-dimethyl-2-heptanol, 2,6-dimethyl-4-heptanol, 3a,4,5,6,7,7a-hexahydro-2,4-dimethyl-4,7-methano[1H]inden-5-ol, 3,7-dimethyl-1,6-nonadien-3-ol, 2,6-dimethyl-2,7-octadien-6-ol (linalool), cis-3,7-dimethyl-2,6-octadien-1-ol (nerol), trans-3,7-dimethyl-2,6-octadien-1-ol (geraniol), 3,7-dimethyl-1,7-octanediol, 3,7-dimethyl-1-octanol (tetrahydrogeraniol), 2,6-dimethyl-2-octanol (tetrahydromyrcenol), 3,7-dimethyl-3-octanol (tetrahydrolinalool), 2,6-dimethyl-7-octen-2-ol (dihydromyrcenol), 3,7-dimethyl-6-octen-1-ol (citronellol), 2,2-dimethyl-3-(3-methylphenyl)-1-propanol, 2,2-dimethyl-3-phenyl-1-propanol, 2-ethoxy-4-methoxymethylphenol, 2-ethyl-4-(2,2,3-trimethyl-3-cyclopenten-1-yl)-2-buten-1-ol, cis-3-hexen-1-ol, 4-(4-hydroxy-3-methoxyphenyl)-2-butanone, 1-hydroxy-2-(1-methyl-1-hydroxyethyl)-5-methylcyclohexane, 3-(hydroxymethyl)-2-nonanone, 4-(4-hydroxy-4-methylpentyl)-3-cyclohexene-1-carboxaldehyde, isoborneol, 3-isocamphylcyclohexanol, 2-isopropenyl-5-methylcyclohexanol (isopulegol), 1-isopropyl-4-methylcyclohex-3-enol (terpinenol), 4-isopropylcyclohexanol, 1-(4-isopropylcyclohexyl) ethanol, 4-isopropylcyclohexylmethanol, 2-isopropyl-5-methylcyclohexanol (menthol), 2-isopropyl-5-methylphenol (thymol), 5-isopropyl-2-methylphenol (carvacrol), 2-(4-methyl-3-cyclohexenyl)-2-propanol (terpineol), 2-(4-methylcyclohexyl)-2-propanol (dihydroterpineol), 4-methoxybenzyl alcohol, 2-methoxy-4-methylphenol, 3-methoxy-5-methylphenol, 1-methoxy-4-propenylbenzene (anethol), 2-methoxy-4-propenylphenol (isoeugenol), 4-methyl-3-decen-5-ol, 2-methyl-6-methylene-7-octen-2-ol (myrcenol), 3-methyl-4-phenyl-2-butanol, 2-(2-methylphenyl) ethanol, 2-methyl-4-phenyl-1-pentanol, 3-methyl-5-phenyl-1-pentanol, 2-methyl-1-phenyl-2-propanol, (1-methyl-2-(1,2,2-trimethylbicyclo[3.1.0]hex-3-ylmethyl)-cyclopropyl) methanol, 3-methyl-4-(2,2,6-trimethylcyclohexen-1-yl)-2-butanol, 2-methyl-4-(2,2,3-trimethyl-3-cyclopenten-1-yl)-2-buten-1-ol, (3-methyl-1-(2,2,3-trimethyl-3-cyclopentenyl)-3-cyclohexen-1-yl) methanol, 3-methyl-5-(2,2,3-trimethyl-3-cyclopenten-1-yl)-4-penten-2-ol, 2-methyl-2-vinyl-5-(1-hydroxy-1-methylethyl)tetrahydrofuran, trans,cis-2,6-nonadienol, 1-nonanol, nopol, 1,2,3,4,4a,5,6,7-octahydro-2,5,5-trimethyl-2-naphthol, 1-octanol, 3,4,5,6,6-pentamethyl-2-heptanol, 2-phenylethanol, 2-phenylpropanol, 3-phenylpropanol (hydrocinnamic alcohol), 3-phenyl-2-propen-1-ol (cinnamic alcohol), 4-(5,5,6-trimethylbicyclo[2.2.1]hept-2-yl) cyclohexan-1-ol, 3,5,5-trimethylcyclohexanol, 2,4,6-trimethyl-4-cyclohexen-1-ylmethanol, 5-(2,2,3-trimethyl-3-cyclopentenyl)-3-methylpentan-2-ol, 3,7,11-trimethyl-2,6,10-dodecatrien-1-ol (farnesol), 3,7,11-trimethyl-1,6,10-dodecatrien-3-ol (nerolidol), 3,5,5-trimethyl-1-hexanol (isononanol), 1-undecanol, 10-undecen-1-ol, vetiverol.

5. An organopolysiloxane according to one of claims 1 to 4,
**characterised.in that**
in an aqueous dispersion of the organopolysiloxane the fragrance alcohol Y-OH is released more quickly at a pH of 3 than at a pH of 9.

6. A textile treatment agent comprising at least one organopolysiloxane according to one of claims 1 to 5.

7. A detergent comprising at least one surfactant and at least one organopolysiloxane according to one of claims 1 to 5.

8. A fragranced fabric rinse comprising at least one organopolysiloxane according to one of claims 1 to 5 in the form of an aqueous dispersion.

9. A personal care product comprising at least one organopolysiloxane according to one of claims 1 to 5.

## Revendications

1. Organopolysiloxane à partir duquel peut être libéré un alcool odoriférant, **caractérisé en ce qu'**il comporte au moins un groupe fonctionnel Z de structure
-N⁺R¹R²-CH₂-C(O)OY A ⁻
dans laquelle
R¹, R² sont choisis, indépendamment l'un de l'autre, parmi des groupes alkyle en C₁-C₃₀ et hydroxyéthyle,
Y est le reste d'un alcool odoriférant Y-OH et
A⁻ est l'anion d'un acide HA physiologiquement acceptable.

2. Organopolysiloxane selon la revendication 1, **caractérisé en ce qu'**il présente une structure correspondant à la formule (I)
R³R⁴R⁵Si-O-CH₂-CH₂-Z (I)
dans laquelle
R³ est un radical polysiloxane relié par un atome d'oxygène,
R⁴ est un groupe alkyle en C₁-C₃₀ ou phényle,
R⁵ = R³ ou R⁴ et
Z a la même signification que dans la revendication 1.

3. Organopolysiloxane selon la revendication 1,
**caractérisé en ce qu'**il présente une structure correspondant à la formule (I)
R⁶-O-[Si(CH₃)₂-O]ₙ-R⁶ (I)
dans laquelle
n = 3-200,
R⁶ = -CH₂-CH₂-N⁺(CH₃)₂-CH₂-C(O)OY A⁻ et
Y et A⁻ ont les mêmes significations que dans la revendication 1.

4. Organopolysiloxane selon l'une quelconque des revendications 1 à 3, **caractérisé en ce que** l'alcool odoriférant Y-OH est choisi dans le groupe constitué par le 4-allyl-2-méthoxyphénol (eugénol), le 3-(2-bornyloxy)-2-méthyl-1-propanol, le 2-tert-butylcyclohexanol, le 4-tert-butylcyclohexanol, l'alcool benzylique, le 1-décanol, le 9-décén-1-ol, le dihydroterpinéol, le 2,4-diméthyl-4-cyclohexén-1-ylméthanol, le 2,4-diméthylcyclohexylméthanol, le 2,6-diméthyl-2-heptanol, le 2,6-diméthyl-4-heptanol, le 3a,4,5,6,7,7a-hexahydro-2,4-diméthyl-4,7-méthano[1H]indén-5-ol, le 3,7-diméthyl-1,6-nonadién-3-ol, le 2,6-diméthyl-2,7-octadién-6-ol (linalool), le *cis*-3,7-diméthyl-2,6-octadién-1-ol (nérol), le *trans*-3,7-diméthyl-2,6-octadién-1-ol (géraniol), le 3,7-diméthyl-1,7-octandiol, le 3,7-diméthyl-1-octanol (tétrahydrogéraniol), le 2,6-diméthyl-2-octanol (tétrahydromyrcénol), le 3,7-diméthyl-3-octanol (tétrahydrolinalool), le 2,6-diméthyl-7-octén-2-ol (dihydromyrcénol), le 3,7-diméthyl-6-octén-1-ol (citronellol), le 2,2-diméthyl-3-(3-méthylphényl)-1-propanol, le 2,2-diméthyl-3-phényl-1-propanol, le 2-éthoxy-4-méthoxyméthylphénol, le 2-éthyl-4-(2,2,3-triméthyl-3-cyclopentén-1-yl)-2-butén-1-ol, le cis-3-hexén-1-ol, la 4-(4-hydroxy-3-méthoxyphényl)-2-butanone, le 1-hydroxy-2-(1-méthyl-1-hydroxyéthyl)-5-méthylcyclohexane, la 3-(hydroxyméthyl)-2-nonanone, le 4-(4-hydroxy-4-méthylpentyl)-3-cyclohexène-1-carboxaldéhyde, l'isobornéol, le 3-isocamphylcyclohexanol, le 2-isopropényl-5-méthylcyclohexanol (isopulégol), le 1-isopropyl-4-méthylcyclohex-3-énol (terpinénol), le 4-isopropylcyclohexanol, le 1-(4-isopropylcyclohexyl)éthanol, le 4-isopropylcyclohexylméthanol, le 2-isopropyl-5-méthylcyclohexanol (menthol), le 2-isopropyl-5-méthylphénol (thymol), le 5-isopropyl-2-méthylphénol (carvacrol), le 2-(4-méthyl-3-cyclohexenyl)-2-propanol (terpinéol), le 2-(4-méthylcyclohexyl)-2-propanol (dihydroterpinéol), l'alcool 4-méthoxybenzylique, le 2-méthoxy-4-méthylphénol, le 3-méthoxy-5-méthylphénol, le 1-méthoxy-4-propénylbenzène (anéthol), le 2-méthoxy-4-propenylphénol (isoeugénol), le 4-méthyl-3-decén-5-ol, le 2-méthyl-6-méthylene-7-octén-2-ol (myrcénol), le 3-méthyl-4-phényl-2-butanol, le 2-(2-méthylphényl)-éthanol, le 2-méthyl-4-phényl-1-pentanol, le 3-méthyl-5-phényl-1-pentanol, le 2-méthyl-1-phényl-2-propanol, le (1-méthyl-2-(1,2,2-triméthylbicyclo[3.1.0]hex-3-ylméthyl)-cyclopropyl)-méthanol, le 3-méthyl-4-(2,2,6-triméthylcyclohexén-1-yl)-2-butanol, le 2-méthyl-4-(2,2,3-triméthyl-3-cyclopenten-1-yl)-2-butén-1-ol, le (3-méthyl-1-(2,2,3-triméthyl-3-cyclopentenyl)-3-cyclohexén-1-yl)-méthanol, le 3-méthyl-5-(2,2,3-triméthyl-3-cyclopentén-1-yl)-4-pentén-2-ol, le 2-méthyl-2-vinyl-5-(1-hydroxy-1-méthyléthyl)-tétrahydrofuranne, le *trans,cis*-2,6-nonadiénol, le 1-nonanol, le nopol, le 1,2,3,4,4a,5,6,7-octahydro-2,5,5-triméthyl-2-naphtol, le 1-octanol, le 3,4,5,6,6-pentaméthyl-2-heptanol, le 2-phényléthanol, le 2-phénylpropanol, le 3-phénylpropanol (alcool hydrocinnamique), le 3-phényl-2-propén-1-ol (alcool cinnamique), le 4-(5,5,6-triméthylbicyclo[2.2.1]hept-2-yl)cyclohexan-1-ol, le 3,5,5-triméthylcyclohexanol, le 2,4,6-triméthyl-4-cyclohexén-1-ylméthanol, le 5-(2,2,3-triméthyl-3-cyclopentényl)-3-méthylpentan-2-ol, le 3,7,11-triméthyl-2,6,10-dodecatrién-1-ol (farnésol), le 3,7,11-triméthyl-1,6,10-dodecatrién-3-ol (nérolidol), le 3,5,5-triméthyl-1-hexanol (isononanol), le 1-undécanol, le 10-undecén-1-ol, le vetivérol.

5. Organopolysiloxane selon l'une quelconque des revendications 1 à 4, **caractérisé en ce que** dans une dispersion aqueuse de l'organopolysiloxane l'alcool odoriférant Y-OH est libéré plus rapidement à un pH de 3 qu'à un pH de 9.

6. Produit de traitement pour textiles, contenant au moins un organopolysiloxane selon l'une quelconque des revendications 1 à 5.

7. Lessive, contenant au moins un tensioactif et au moins un organopolysiloxane selon l'une quelconque des revendications 1 à 5.

8. Rinçage parfumé, contenant au moins un organopolysiloxane selon l'une quelconque des revendications 1 à 5, sous forme d'une dispersion aqueuse.

9. Produit de soin pour le corps, contenant au moins un organopolysiloxane selon l'une quelconque des revendications 1 à 5.
